# EUROPEAN PATENT APPLICATION

(11) **EP 3 607 955 A1**
(43) Date of publication of application: **12.02.2020**
(21) Application number: 19179032.8
(22) Date of filing: 30.01.2013
(51) Int. Cl.: A61K 31/795, A61P 7/04

(54) **NON-ANTICOAGULANT SULFATED OR SULFONATED SYNTHETIC POLYMERS**

(30) Priority: 30.01.2012 US 201261592554 P
(62) Divisional of application: 13702878.3
(71) Applicant: Baxalta GmbH, 6300 Zug (CH); Baxalta Incorporated, Bannockburn, IL 60015 (US)
(72) Inventor: Dockal, Michael, 1150 Vienna (AT); Scheiflinger, Fritz, 1090 Vienna (AT); Knappe, Sabine, 1060 Vienna (AT); Till, Susanne, 1050 Vienna (AT); Hai, Ton, Round Lake, IL Illinois 60073 (US); Sanders, Paul, Greendale, WI Wisconsin 53129 (US)
(74) Representative: Hoffmann Eitle

(57) **Abstract**

The present invention provides pharmaceutical formulations including a non-anticoagulant, non-saccharide polymer that with at least one sulfate or sulfonate moiety. The pharmaceutical formulations of the invention are of use to improve blood clotting in a subject. Also provided are useful analytical methods utilizing these polymers, to query the dynamics of blood clotting *in vitro*.

## Description

### CROSS-REFERENCES TO RELATED APPLICATIONS

The present application claims the benefit of U.S. Provisional Application No. 61/592,554, filed January 30, 2012, the content of which is expressly incorporated herein by reference in its entirety for all purposes.

### BACKGROUND OF THE INVENTION

Normal blood coagulation is a complex physiological and biochemical process involving activation of a coagulation factor cascade leading to fibrin formation and platelet aggregation along with local vasoconstriction (reviewed by Davie, et al., Biochemistry, 30:10363, 1991). The clotting cascade is composed of an "extrinsic" pathway thought to be the primary means of normal coagulation initiation and an "intrinsic" pathway contributing to an expanded coagulation response. The normal response to a bleeding insult involves activation of the extrinsic pathway. Activation of the extrinsic pathway initiates when blood comes in contact with tissue factor (TF), a cofactor for Factor VII that becomes exposed or expressed on tissues following insult. TF forms a complex with FVII that facilitates the production of FVIIa. FVIIa then associates with TF to convert FX to the serine protease FXa, which is a critical component of the prothrombinase complex. The conversion of prothrombin to thrombin by the FXa/FVa/calcium/phospholipid complex stimulates the formation of fibrin and activation of platelets, all of which is essential to normal blood clotting. Normal hemostasis is further enhanced by intrinsic pathway Factors IXa and VIIIa, which also convert FX to FXa.

Blood clotting is inadequate in bleeding disorders, which may be caused by congenital coagulation disorders, acquired coagulation disorders, or hemorrhagic conditions induced by trauma. Bleeding is one of the most serious and significant manifestations of disease, and may occur from a local site or be generalized. Localized bleeding may be associated with lesions and may be further complicated by a defective hemostatic mechanism. Congenital or acquired deficiencies of any of the coagulation factors may be associated with a hemorrhagic tendency. Congenital coagulation disorders include hemophilia, a recessive X-linked disorder involving a deficiency of coagulation Factor VIII (hemophilia A) or Factor IX (hemophilia B) and von Willebrand disease, a rare bleeding disorder involving a severe deficiency of von Willebrand Factor. Acquired coagulation disorders may arise in individuals without a previous history of bleeding as a result of a disease process. For example, acquired coagulation disorders may be caused by inhibitors or autoimmunity against blood coagulation factors, such as Factor VIII, von Willebrand Factor, Factors IX, V, XI, XII and XIII; or by hemostatic disorders such as caused by liver disease, which may be associated with decreased synthesis of coagulation factors. Coagulation factor deficiencies are typically treated by factor replacement which is expensive, inconvenient (intravenous), and not always effective.

The treatment of blood clotting disorders including hemophilia (hem), severe von Willebrand (svWD) disease, and severe Factor VII deficiency are typically treated with coagulation factors such as Factor VIII (used to treat hem and svWD). The downside associated with treatments centered on administering coagulation factors include their high cost, the necessity of IV administration of these proteins, and the generation of antibodies that neutralize the effects of the coagulation factors. Up to approximately 20% of patients receiving chronic factor replacement therapy may generate neutralizing antibodies to replacement factors.

Thus, there remains a need for new therapeutic approaches for treating bleeding disorders. A single pharmaceutical agent that is safe, convenient and effective in a broad range of bleeding disorders would favorably impact clinical practice.

### SUMMARY OF THE INVENTION

The present invention provides compositions and methods for treating bleeding disorders using non-anticoagulant sulfated or sulfonated synthetic polymers (NASSPs) as procoagulants. NASSPs can be administered as single agents, or in combination with one another, or with other hemostatic agents. In particular, the use of NASSPs in treatment of bleeding disorders, including congenital coagulation disorders, acquired coagulation disorders, and trauma induced hemorrhagic conditions is provided.

The present invention provides numerous advantages. For example, polymers as base molecules for sulfation or sulfonation are structurally well defined, of low molecular weight and are commercially available. Furthermore, chemical sulfation or sulfonation of polymers, or the de novo synthesis of sulfated or sulfonated polymers from monomers or unsulfated polymers, allows adjustment of sulfation or sulfonation degree and sulfation or sulfonation pattern allowing for the characterization of the structure activity relationship of the sulfated or sulfonated synthetic polymers. In an exemplary embodiment, the invention provides an oral dosage form incorporating one or more sulfated or sulfonated synthetic polymer of the invention, which improves patient care through increased ease of administration and patient compliance.

In one aspect, the invention provides a sulfated or sulfonated synthetic polymer with the ability to enhance coagulation of mammalian blood *in vivo* and/or *in vitro.* In various embodiments, the sulfated or sulfonated synthetic polymer has procoagulant activity. In various aspects the procoagulant activity of the sulfated or sulfonated synthetic polymer is of sufficient magnitude that it is measurable using a standard assay, *e.g*., the Thrombin Generation Assay (TGA).

In various embodiments, the invention provides a non-saccharide sulfated or sulfonated synthetic polymer having the formula selected from: 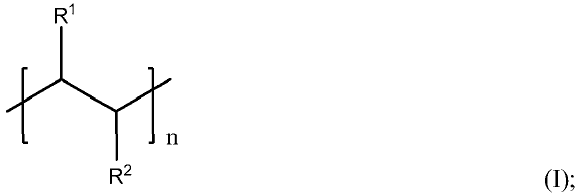 and 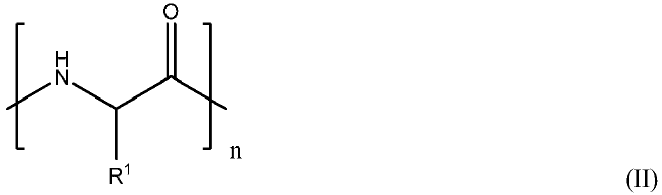 wherein R¹ is selected from H, substituted or unsubsituted alkyl and sulfonated aryl. R² is selected from H, substituted or unsubstituted alkyl and sulfonate, such that at least one of R¹ and R² is or includes a sulfonate or sulfate group (*e.g*., SO₃⁻M⁺, or SO₃H; M⁺ is an inorganic or organic cation). In an exemplary embodiment, only one of R¹ and R² is or includes a sulfonate or sulfate moiety. The index n represents an integer greater than 0, which signifies the number of repeated subunits in the polymer. In exemplary embodiments, n is selected to provide a polymer with a molecular weight from about 7 kDa to about 300 kDa, for example, from about 9 kDa to about 200 kDa, *e.g*., from about 11 kDa to about 100kDa. Exemplary sulfated or sulfonated synthetic polymers of the invention provide a subject administered one of these polymers a therapeutically relevant procoagulant effect. Exemplary compounds of the invention also exert an anticoagulant effect upon administration to a subject. In various embodiments, the polymers of the invention do not induce a degree of anticoagulant effect sufficient to significantly, or to entirely offset the procoagulant effect of the polymer. In an exemplary embodiment, the compounds of the invention have a procoagulant effect at concentrations of from about 0.1 µg/mL to about 300 µg/mL, *e.g*., about 1 µg/mL to about 100 µg/mL, *e*.*g*., about 3µg/mL to about 30 µg/mL.

In various embodiments, R¹ is: 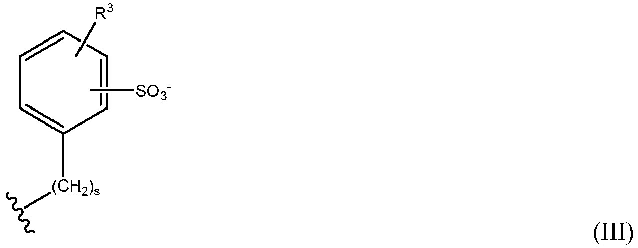 in which R³ is selected from H and OR⁴. The index s is 0, 1, 2, 3 or higher. R⁴ is selected from H, and substituted or unsubstituted alkyl. In various embodiments in which R¹ is the substituted phenyl group (III), R² is H.

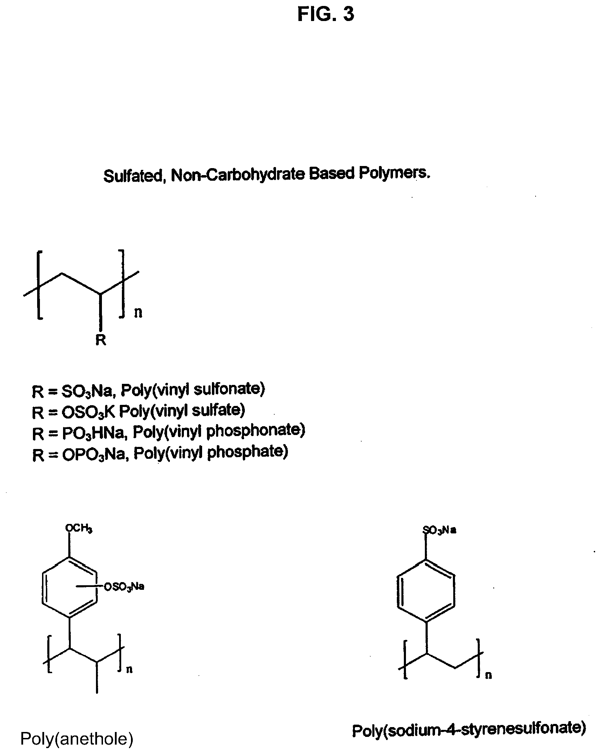

Exemplary polymers according to the present invention are set forth in **FIG. 3****.**

In other embodiments the NASSP of the invention decreases blood clotting time when tested in the TFPI- dilute prothrombin time clotting assay. In various embodiments, the invention reverses the anticoagulant effect of exogenous full-length TFPI (flTFPI) in human plasma. In various embodiments, the compound of the invention does not interfere with the action of TFPI160. In various embodiments, the NASSP of the invention interacts with the C-terminus of TFPI to provide procoagulant activity.

In an exemplary embodiment, the composition is of use in a method for treating a subject in need of enhanced blood coagulation. The method comprises administering a therapeutically effective amount of a composition comprising a non-anticoagulant, non-saccharide sulfated or sulfonated synthetic polymer of the invention (NASSP) to the subject.

In certain embodiments, the invention provides a method for treating a subject having a bleeding disorder comprising administering a therapeutically effective amount of a composition comprising a NASSP of the invention to the subject.

In certain embodiments, a NASSP of the invention is administered to a subject to treat a bleeding disorder selected from the group consisting of hem A, hem B, von Willebrand disease, idiopathic thrombocytopenia, a deficiency of one or more coagulation factors (*e.g*., Factor XI, Factor XII, prekallikrein, and HMWK), a deficiency of one or more coagulation factors associated with clinically significant bleeding (*e*.*g.*, Factor V, Factor VII, Factor VIII, Factor IX, Factor X, Factor XIII, Factor II (hypoprothrombinemia), and von Willebrands Factor, a vitamin K deficiency, a disorder of fibrinogen (*e.g*., afibrinogenemia, hypofibrinogenemia, and dysfibrinogenemia), an alpha2-antiplasmin deficiency, and excessive bleeding such as caused by liver disease, renal disease, thrombocytopenia, disorders with decreased platelet number, hematomas, internal hemorrhage, hemarthroses, surgery, trauma, hypothermia, menstruation, and pregnancy.

In certain embodiments, a NASSP is administered to a subject to treat a congenital coagulation disorder or an acquired coagulation disorder caused by a blood factor deficiency. In various embodiments, the blood factor deficiency is caused by deficiencies of one or more factors (*e.g*., Factor V, Factor VII, Factor VIII, Factor IX, Factor XI, Factor XII, Factor XIII, and von Willebrand Factor).

In exemplary embodiments, the NASSP of the invention is co-administered with one or more different NASSPs and/or in combination with one or more other therapeutic agents. In an exemplary embodiment, the NASSP is co-administered with one or more NASP. Examples of useful NASPs include those disclosed in commonly-owned, co-pending U.S. Patent Application No. 61/592,549, bearing Attorney Docket No. 008073-5034-PR. In certain embodiments, a subject having a bleeding disorder is administered a therapeutically effective amount of a composition comprising a NASSP of the invention in combination with another therapeutic agent. For example, the subject may be administered a therapeutically effective amount of a composition comprising a NASSP of the invention and one or more factors. Exemplary factors of use in this embodiment include, without limitation, Factor XI, Factor XII, prekallikrein, HMWK, Factor V, Factor VII, Factor VIII, Factor IX, Factor X, Factor XIII, Factor II, Factor VIIa, and von Willebrand Factor. Treatment may further comprise administering a procoagulant such as thrombin; an activator of the intrinsic coagulation pathway, including Factor Xa, Factor IXa, Factor XIa, Factor XIIa, and VIIIa, prekallekrein, and high-molecular weight kininogen (HMWK); or an activator of the extrinsic coagulation pathway, including tissue factor, Factor VIIa, Factor Va, and Factor Xa. Therapeutic agents used to treat a subject having a bleeding disorder can be administered in the same or different compositions and concurrently, before, or after administration of a NASSP of the invention.

In various aspects, the invention provides a method for reversing the effects of an anticoagulant in a subject. The method includes administering a therapeutically effective amount of a composition comprising a non-anticoagulant sulfated or sulfonated synthetic polymer (NASSP) of the invention to the subject. In certain embodiments, the subject may have been treated with an anticoagulant including, but not limited to, heparin, a coumarin derivative, such as warfarin or dicumarol, tissue factor pathway inhibitor (TFPI), antithrombin III, lupus anticoagulant, nematode anticoagulant peptide (NAPc2), active-site blocked factor VIIa (Factor VIIai), Factor IXa inhibitors, Factor Xa inhibitors, including fondaparinux, idraparinux, DX-9065a, and razaxaban (DPC906), inhibitors of Factors Va and VIIIa, including activated protein C (APC) and soluble thrombomodulin, thrombin inhibitors, including hirudin, bivalirudin, argatroban, and ximelagatran. In certain embodiments, the anticoagulant in the subject may be an antibody that binds a coagulation factor, including but not limited to, an antibody that binds to Factor V, Factor VII, Factor VIII, Factor IX, Factor X, Factor XIII, Factor II, Factor XI, Factor XII, von Willebrand Factor, prekallikrein, or high molecular weight kininogen (HMWK).

In certain embodiments, a NASSP of the invention can be co-administered with one or more different NASSPs and/or in combination with one or more other therapeutic agents (*e.g*., NASP or a coagulation factor) for reversing the effects of an anticoagulant in a subject. For example, the subject may be administered a therapeutically effective amount of a composition comprising a NASSP of the invention and one or more NASP and/or factors selected from the group consisting of Factor XI, Factor XII, prekallikrein, high molecular weight kininogen (HMWK), Factor V, Factor VII, Factor VIII, Factor IX, Factor X, Factor XIII, Factor II, Factor VIIa, and von Willebrand Factor. Treatment may further comprise administering a procoagulant, such as an activator of the intrinsic coagulation pathway, including Factor Xa, Factor IXa, Factor XIa, Factor XIIa, and VIIIa, prekallekrein, and high-molecular weight kininogenHMWK; or an activator of the extrinsic coagulation pathway, including tissue factor, Factor VIIa, Factor Va, and Factor Xa. Therapeutic agents used in combination with a NASSP of the invention to reverse the effects of an anticoagulant in a subject can be administered in the same or different compositions and concurrently, before, or after administration of the NASSP of the invention.

In another aspect, the invention provides a method for treating a subject undergoing a surgical or invasive procedure in which improved blood clotting is desirable. The method includes administering a therapeutically effective amount of a composition comprising a non-anticoagulant sulfated or sulfonated synthetic polymer (NASSP) of the invention to the subject. In certain embodiments, the NASSP of the invention can be co-administered with one or more different NASSPs and/or in combination with one or more other therapeutic agents (*e.g*., NASP or a coagulation factor). For example, in addition to the NASSP of the invention, the subject may be administered a therapeutically effective amount of one or more factors selected from the group consisting of Factor XI, Factor XII, prekallikrein, high molecular weight kininogen (HMWK), Factor V, Factor VII, Factor VIII, Factor IX, Factor X, Factor XIII, Factor II, Factor VIIa, and von Willebrand Factor. Treatment may further comprise administering a procoagulant, such as an activator of the intrinsic coagulation pathway, including Factor Xa, Factor IXa, Factor XIa, Factor XIIa, and VIIIa, prekallikrein, and HMWK; or an activator of the extrinsic coagulation pathway, including tissue factor, Factor VIIa, Factor Va, and Factor Xa. Therapeutic agents used to treat a subject undergoing a surgical or invasive procedure can be administered in the same or different compositions and concurrently, before, or after administration of the NASSP of the invention.

In another aspect, the invention provides a method of inhibiting TFPI activity in a subject. The method includes administering a therapeutically effective amount of a composition comprising a NASSP of the invention to the subject.

In another aspect, the invention provides a method of inhibiting TFPI activity in a biological sample. The method includes combining the biological sample (*e.g*., blood or plasma) with a sufficient amount of a non-anticoagulant sulfated or sulfonated synthetic polymer (NASSP) of the invention to inhibit TFPI activity.

In another aspect, the invention provides a composition comprising a NASSP of the invention. In certain embodiments, the NASSP is a sulfated or sulfonated synthetic polymer in which the base polymer is selected from polyvinyl, polystyrene or polyanethole. In certain embodiments, the composition further comprises a pharmaceutically acceptable excipient. In certain embodiments, the composition further comprises one or more different NASSPs, and/or one or more therapeutic agents, and/or reagents. For example, the composition may further comprise one or more factors selected from the group consisting of Factor XI, Factor XII, prekallikrein, high molecular weight kininogen (HMWK), Factor V, Factor VII, Factor VIII, Factor IX, Factor X, Factor XIII, Factor II, and von Willebrands Factor, tissue factor, Factor VIIa, Factor Va, and Factor Xa, Factor IXa, Factor XIa, Factor XIIa, and VIIIa; and/or one or more composition selected from the group consisting of APTT reagent, thromboplastin, fibrin, Russell's viper venom, micronized silica particles, ellagic acid, sulfatides, and kaolin.

These and other embodiments of the subject invention will readily occur to those of skill in the art in view of the disclosure herein.

### BRIEF DESCRIPTION OF THE DRAWINGS

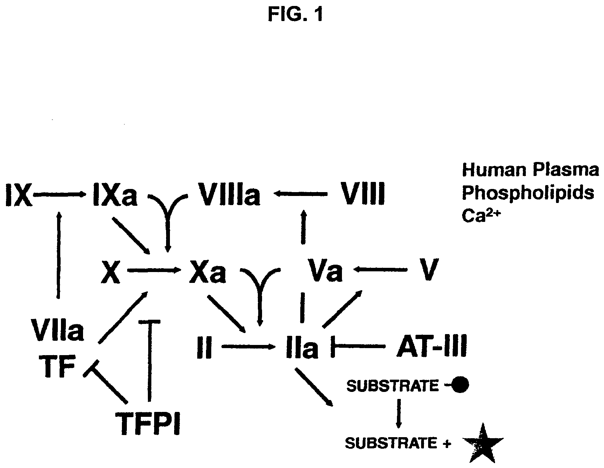

**FIG. 1** is a flowchart showing the generation of thrombin.
**FIG 2** shows a calibrated automatic thrombogram (CAT).
**FIG. 3** displays exemplary compounds of the invention and of use in the methods of the invention.

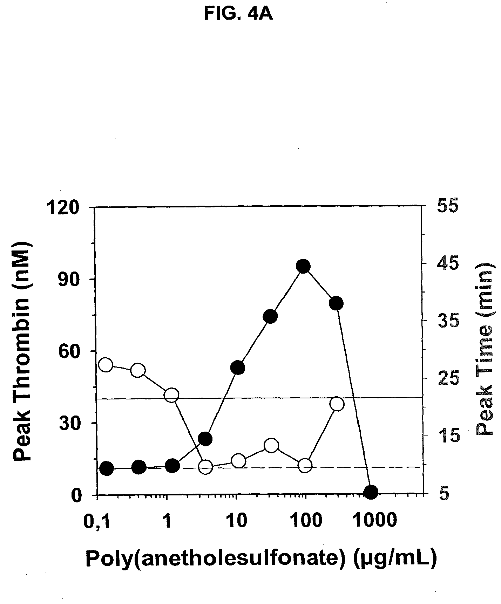

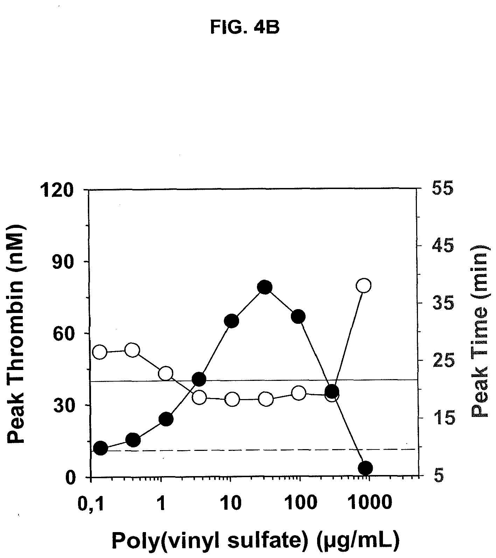

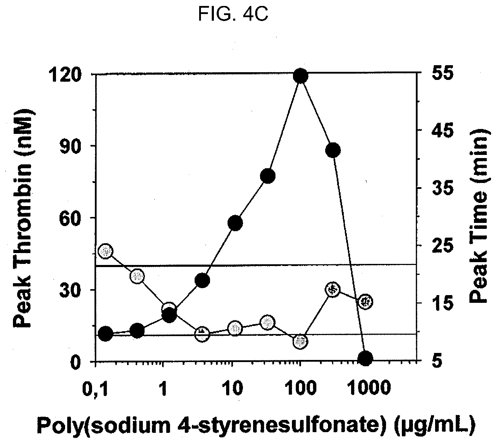

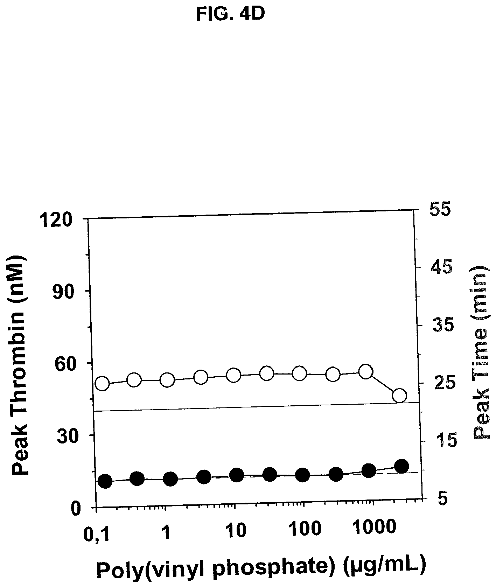

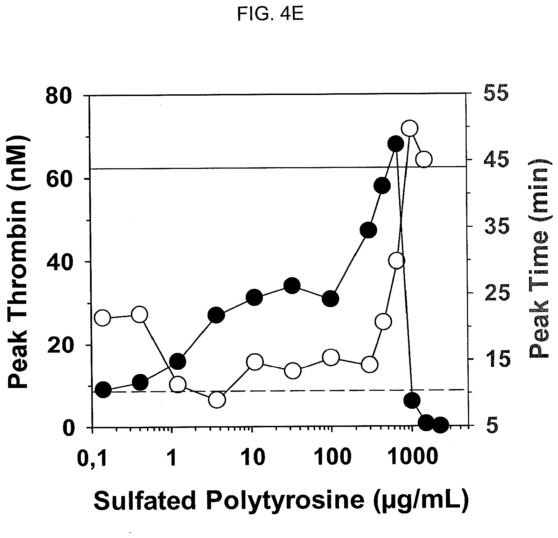

**FIG. 4A-E** displays procoagulant activity and procoagulant window of sulfated, sulfonated and phosphorylated polymers. CAT (peak thrombin, colored circles; time to peak, white circles) of (**FIG. 4A**) Poly(anetholesulfonic acid) sodium salt, (**FIG. 4B**) Poly(sodium 4-styrenesulfonate), (**FIG. 4C**) Poly(vinyl sulfate) potassium salt and (**FIG. 4D**) Poly(vinyl phosphoric acid) sodium salt in FVIII-inhibited plasma as a model for antibody-induced hemophilia. Thrombin generation is triggered by recalcification and low tissue factor (1 pM). Sulfated or sulfonated polymers start to increase peak thrombin and reduce time to peak at concentrations of about 0.3 µg/mL, indicative of their procoagulant activity. At about 30 to 100 µg/mL NASSP added to FVIII-inhibited plasma thrombin generation is optimal and exceeds the thrombin generation of a human normal plasma pool (black reference line: peak thrombin of a human normal plasma pool). At concentrations higher than about 100 µg/mL peak thrombin decreases and peak time increases which is indicative of the onset of their anticoagulant activities. At about 300 µg/mL peak thrombin is still above the starting level of FVIII-inhibited plasma (dashed reference line: peak thrombin of a FVIII-inhibited patient plasma) opening up a large procoagulant window for therapeutic efficacy. In contrast to the procoagulant activities of NASSPs, poly(vinyl phosphoric acid) sodium salt does not improve thrombin generation of FVIII-inhibited plasma. (**FIG. 4E**) Procoagulant activity and procoagulant window of sulfated polytryosine. CAT (peak thrombin , colored circles; time to thrombin peak, white circles) of sulfated polytyrosine in FVIII -inhibited plasma as a model for hemophilia. Thrombin generation is triggered by recalcification and low tissue factor (1 pM). Sulfated polytyrosine starts to increase peak thrombin and reduce time to peak at concentrations of about 0.3 µg/mL, indicative of its procoagulant activity. Starting at about 5 µg/mL, sulfated polytyrosine added to FVIII-inhibited plasma thrombin generation is optimal and significantly improves the thrombin generation of a human normal plasma pool (black reference line: peak thrombin of a human normal plasma pool). At a concentration of about 700 µg/mL peak thrombin is decreasing and peak time is increasing which is indicative of the onset of its anticoagulant activities. Only at about 1 mg/mL peak thrombin reaches the starting level of FVIII-inhibited plasma (dashed reference line: peak thrombin of a FVIII-inhibited normal plasma) opening up a large procoagulant window for therapeutic efficacy.

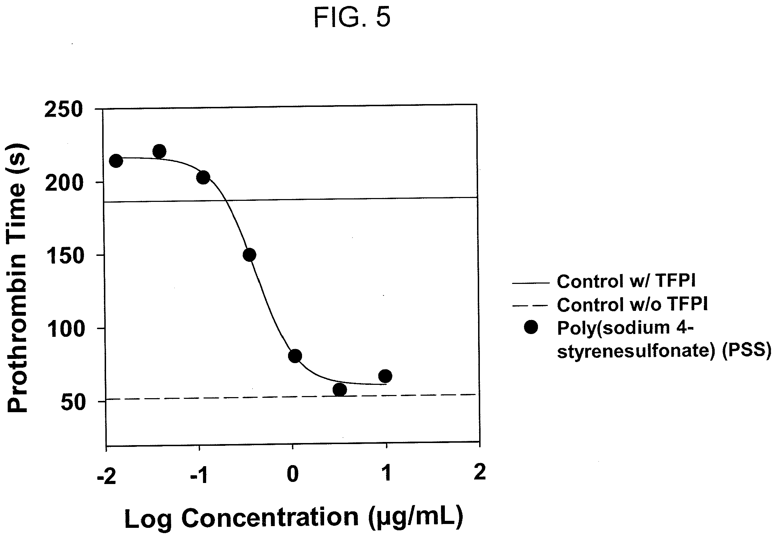

**FIG. 5** shows a plot from a TFPI-dPT assay of poly(sodium-4-styrenesulfonate, Mw = 70 kDa) (PSS). The assay was run in normal human plasma with human full-length TFPI (huflTFPI) at 0.5 µg/mL plasma concentration, and CaCl2 at 8.3 mM. PSS inhibits human flTFPI with an EC50 of 0.42 µg/mL.

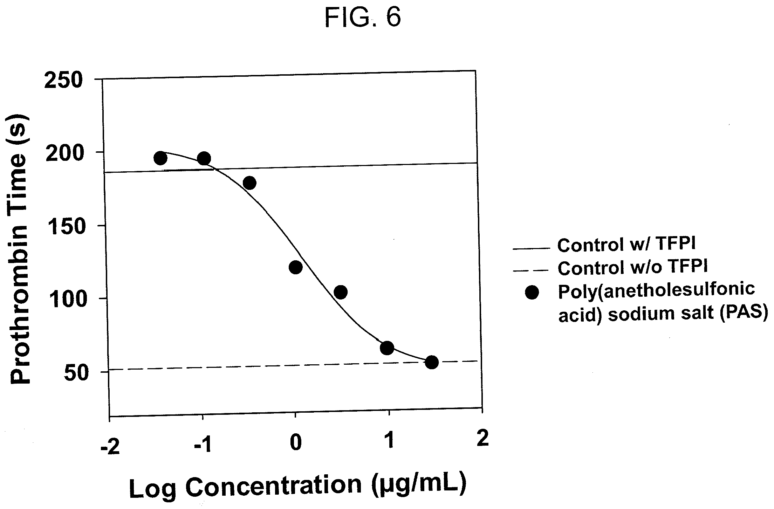

**FIG. 6** shows a plot from a TFPI-dPT assay of poly(anetholesulfonic acid, sodium salt), Mw = 9-11 kDa) (PAS). The assay was run in normal human plasma with huflTFPI at 0.5 µg/mL plasma concentration, and CaC12 at 8.3 mM. PAS inhibits human flTFPI with an EC50 of 1.27 µg/mL.

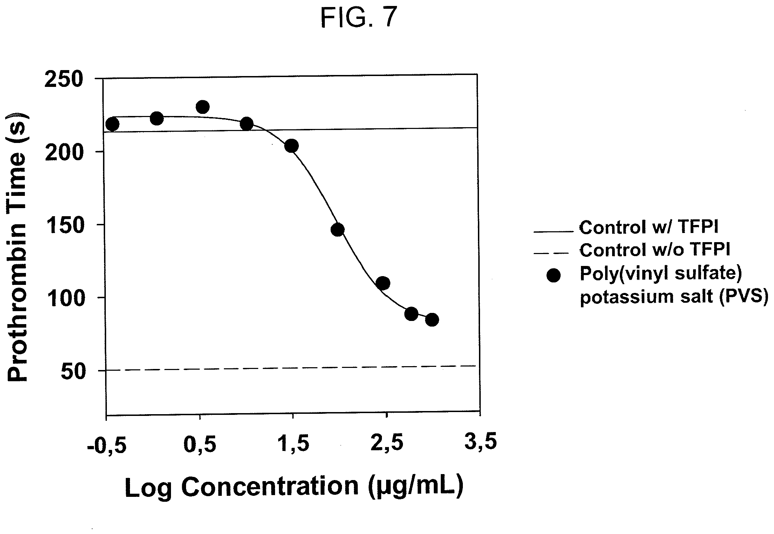

**FIG. 7** shows a plot from a TFPI-dPT assay of poly(vinylsulfate, potassium salt), Mw = 170 kDa) (PVS). The assay was run in normal human plasma with huflTFPI at 0.5 µg/mL plasma concentration, and CaCl2 at 8.3 mM. PVS inhibits human flTFPI with an EC50 of 94.3 µg/mL.

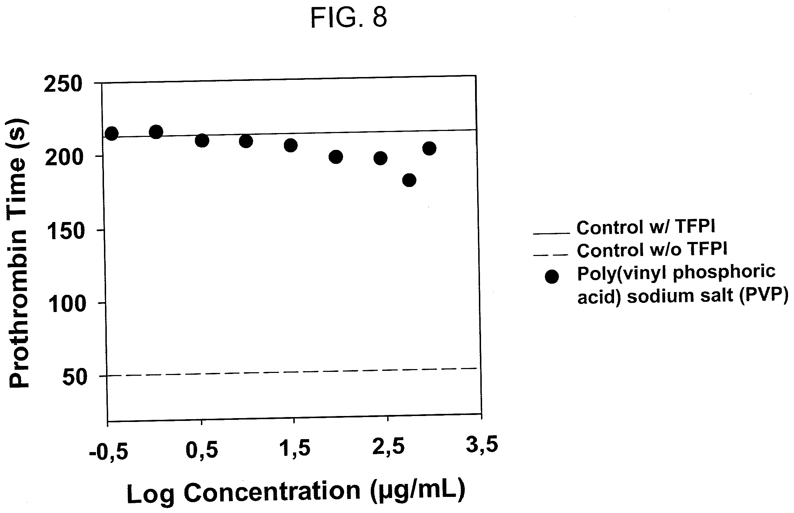

**FIG. 8** shows a plot from a TFPI-dPT assay of poly(vinylphosphoric acid, sodium salt), Mw > 200 kDa) (PVP). The assay was run in normal human plasma with huflTFPI at 0.5 µg/mL plasma concentration, and CaCl2 at 8.3 mM. PVP shows no inhibition of human flTFPI.

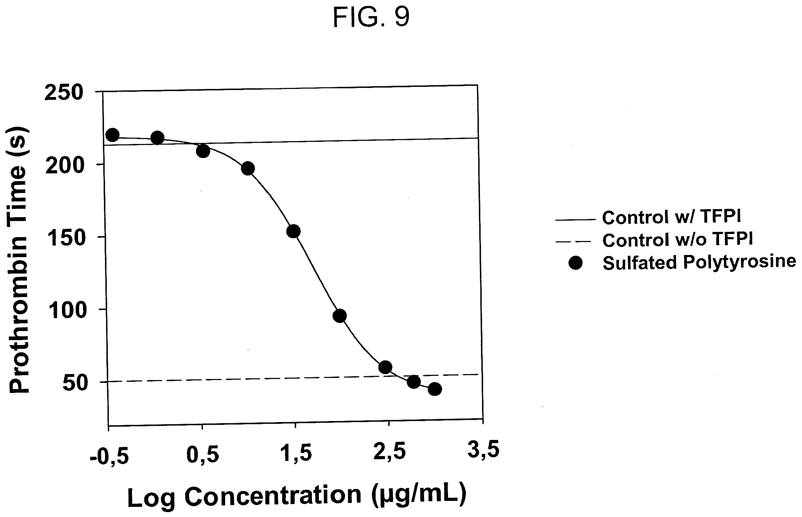

**FIG. 9** shows a plot from a TFPI-dPT assay of sulfated poly(tyrosine), (PVP). The assay was run in normal human plasma with huflTFPI at 0.5 µg/mL plasma concentration and CaCl2 at 8.3 mM. PT inhibits human flTFPI with an EC50 of 51.7 µg/mL.

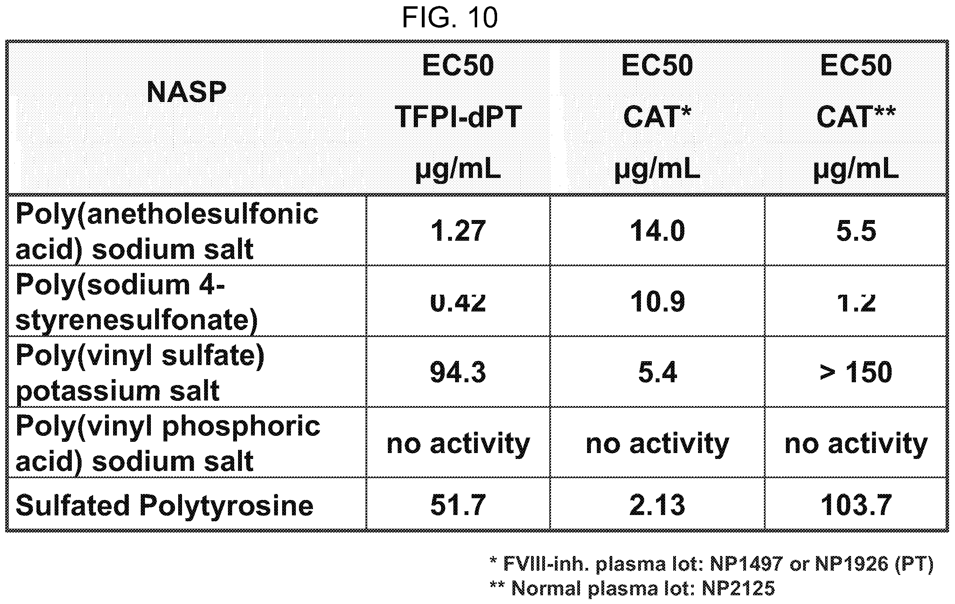

**FIG. 10** is a tabulation comparing EC50 values derived from CAT assays and TFPI-dPT assays of NASSPs of the invention..

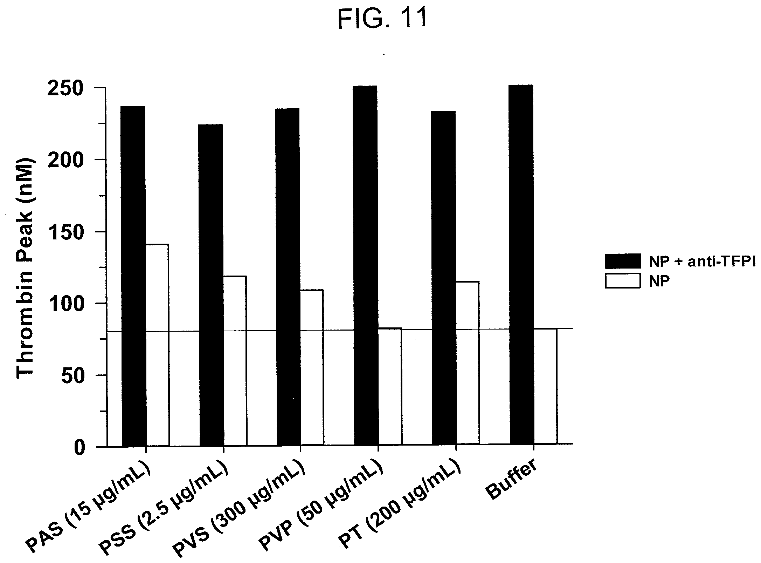

**FIG. 11** shows a CAT assay with NASSPs of the invention in normal human plasma. NASSPs improve thrombin generation at the indicated concentrations in contrast to poly(vinylphosphoric acid, sodium salt), (PVP) and buffer (white bars; reference line). Improvement of thrombin generation by NASSPs was not observed upon blockage of TFPI by a polyclonal anti-TFPI antibody (AF2974, colored bars). This suggests that TFPI is necessary for the procoagulant action of NASSPs.

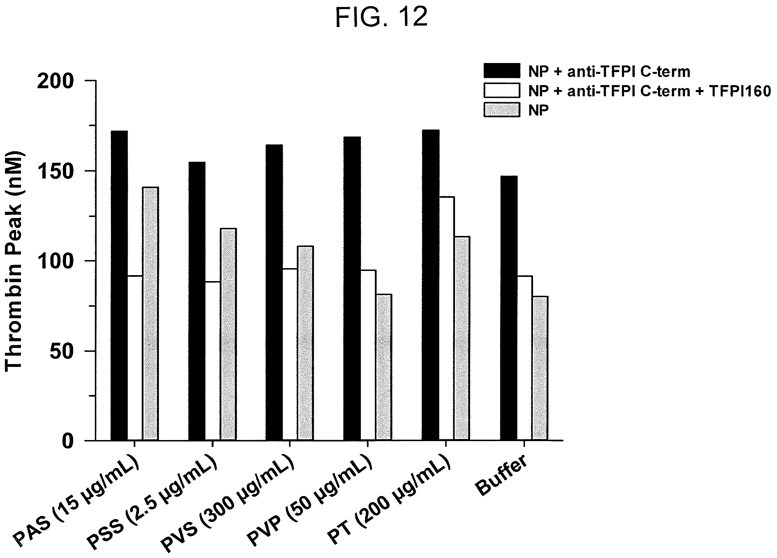

**FIG. 12** shows a CAT assay with NASSPs of the invention in normal human plasma. NASSPs improve thrombin generation at the indicated concentrations in contrast to poly(vinylphosphoric acid, sodium salt), (PVP) and buffer (grey bars). Improvement of thrombin generation by NASSPs is abrogated by blockage of TFPI using an anti-TFPI antibody directed against the C-terminus of TFPI (colored bars). Replacement with a TFPI fragment (aa 1-160) does not recover the procoagulant effect of NASSPs (white bars) suggesting that the NASSPs of the invention act on the C-terminus of TFPI. Some procoagulant effect is observed for sulfated poly(tyrosine) in the presence of TFPI60.
**FIG. 13A-B** is a matrix table showing exemplary combinations of certain types of NASSPs (based on their base polymer) with additional agents.
**FIG. 14A-B** is a matrix table showing exemplary dosage ranges for the respective NASSP in each of the combinations identified in **FIG. 13A-B****.**
**FIG. 15** is a table showing exemplary dosage ranges for certain additional agents used in combination with the NASSPs (such as, for example, the combinations shown in **FIG. 13A-B** and **FIG. 14A-B**).
FIG. **16A-B** shows the anticoagulant properties of exemplary NASSPs of the invention. Exemplary NASSPs have anticoagulant properties at higher concentrations than that at which they show pro-coagulant activity.

### DETAILED DESCRIPTION OF THE INVENTION

### Introduction

Blood clotting disorders including hemophilia, *e.g*., hem A and Hem B, severe von Willebrand disease (svWD), and severe Factor VII (FVII) deficiency have typically been treated by Factor replacement, *e.g*., Factor VIII for hem A and svWD, Factor IX for hem B, and Factor VII(a) for FVII-deficiency and others (reviewed in Bishop, et al. (2004) Nat. Rev. Drug Discov., 3:684-694; Carcao, et al. (2004) Blood Rev., 18:101-113; Roberts, et al. (2004) Anesthesiology 100:722-730; and Lee (2004) Int. Anesthesiol. Clin., 42:59-76). While such therapies are often effective, characteristics limiting utility include high cost, inconvenience (*i.e*., intravenous administration), and neutralizing antibody generation (Bishop, *et al*., *supra*; Carcao, *et al*., *supra*; Roberts, *et al*., *supra*; Lee, *supra*; and Bohn, et al. (2004) Haemophilia 10 Suppl., 1:2-8). While FVIIa is increasingly utilized in various bleeding disorders (Roberts, *et al*., *supra*), alternative single compound procoagulant therapies devoid of the aforementioned constraints and disadvantages, and with broad application are of interest.

One general approach to improving hemostasis in individuals with bleeding disorders is to improve the initiation of clotting by upregulating the extrinsic pathway of blood coagulation. While the intrinsic and extrinsic pathways of coagulation contribute to thrombin generation and fibrin clot formation (Davie, et al. (1991) Biochemistry, 30:10363-10370), the extrinsic-or tissue factor (TF) mediated-path is critical for initiation, and contributes to propagation of coagulation *in vivo* (Mann (2003) Chest, 124(3 Suppl): 1S-3S; Rapaport, et al. (1995) Thromb. Haemost., 74:7-17). One potential mechanism for upregulating extrinsic pathway activity is the attenuation of Tissue Factor Pathway Inhibitor (TFPI). TFPI is a Kunitz-type proteinase inhibitor of FVIIa/TF that provides tonic downregulation of extrinsic pathway activation (see Broze (1992) Semin. Hematol., 29:159-169; Broze (2003) J. Thromb. Haemost., 1:1671-1675; and Johnson, et al. (1998) Coron. Artery Dis., 9(2-3):83-87 for review). Indeed, heterozygous TFPI deficiency in mice can result in exacerbation of thrombus formation (Westrick, et al. (2001) Circulation, 103:3044-3046), and TFPI gene mutation is a risk factor for thrombosis in humans (Kleesiek, et al. (1999) Thromb. Haemost., 82:1-5). Regulating clotting in hemophilia via the targeting of TFPI was described by Nordfang, *et al*. and Wun, *et al*., who showed that anti-TFPI antibodies could shorten the coagulation time of hemophilic plasma (Nordfang, et al. (1991) Thromb. Haemost., 66:464-467; Welsch, et al. (1991) Thromb. Res., 64:213-222) and that anti-TFPI IgG improved the bleeding time of rabbits that were Factor VIII-deficient (Erhardtsen, et al. (1995) Blood Coagul. Fibrinolysis, 6:388-394).

As described herein, certain sulfated or sulfonated synthetic polymers of the invention (NASSP) inhibit anticoagulant activity at lower concentrations than concentrations at which the NASSP exhibits significant anticoagulation. Such molecules are of use in settings where clot formation is compromised.

Before describing the present invention in detail, it is to be understood that this invention is not limited to particular formulations or process parameters. It is also to be understood that the terminology used herein is for the purpose of describing particular embodiments of the invention only, and is not intended to be limiting.

Although a number of methods and materials similar or equivalent to those described herein can be used in the practice of the present invention, the preferred materials and methods are described herein.

### Definitions

In describing the present invention, the following terms will be employed, and are intended to be defined as indicated below.

As used in this specification and the appended claims, the singular forms "a", "an" and "the" include plural referents unless the content clearly dictates otherwise. Thus, for example, reference to "a NASSP" includes a mixture of two or more such agents, and the like.

A "NASSP" as used herein refers to a sulfated or sulfonated synthetic polymer that exhibits anticoagulant activity in an activated partial thromboplastin time (aPTT) clotting assay that is no more than one-third, and preferably less than one-tenth, the anticoagulant increase in clotting time activity of unfractionated heparin (*e.g*., as measured by increase per µg/mL). NASSPs of the invention may be synthesized de novo and may range in molecular weight from about 10 daltons to about 1,000,000 daltons, such as for example, from about 100 daltons to about 900,000 daltons, such as from about 500 daltons to about 500,000 daltons, such as from about 1000 daltons to about 250,000 daltons, including about 5000 daltons to about 150,000 daltons. NASSPs may range in average molecular weight from about 10 daltons to about 500,000 daltons, such as from about 100 daltons to about 300,000 daltons, such as from about 1000 daltons to about 250,000 daltons, including about 1000 daltons to about 150,000 daltons. NASSPs of the invention may be used in the methods of the invention, inter alia for improving hemostasis in treating bleeding disorders, particularly those associated with deficiencies of coagulation factors or for reversing the effects of anticoagulants. The ability of NASSPs of the invention to promote clotting and reduce bleeding is readily determined using various *in vitro* global hemostatic and clotting assays (*e.g*., TFPI-dPT and CAT assays) and *in vivo* bleeding models (*e.g*., tail transection, transverse cut, whole blood clotting time, or cuticle bleeding time determination in hemophilic mice or dogs). See, *e.g*., PDR Staff. Physicians' Desk Reference. 2004, Anderson, et al. (1976) Thromb. Res., 9:575-580; Nordfang, et al. (1991) Thromb Haemost., 66:464-467; Welsch, et al. (1991) Thrombosis Research, 64:213-222; Broze, et al. (2001) Thromb Haemost, 85:747-748; Scallan, et al. (2003) Blood, 102:2031-2037; Pijnappels, et al. (1986) Thromb. Haemost., 55:70-73; and Giles, et al. (1982) Blood, 60:727-730.

A "procoagulant" is used herein in its conventional sense to refer to any factor or reagent capable of initiating or accelerating clot formation. Exemplary procoagulants include a NASSP of the invention, coagulation factor or reagent capable of initiating or accelerating clot formation. Exemplary procoagulants of use in a composition of the invention include any activator of the intrinsic or extrinsic coagulation pathways, such as a NASSP, a coagulation factor selected from the group consisting of Factor Xa, Factor IXa, Factor XIa, Factor XIIa, and Factor VIIIa, prekallikrein, HMWK, tissue factor, Factor VIIa, and Factor Va. Other reagents that promote clotting include kallikrein, APTT initiator (*i.e*., a reagent containing a phospholipid and a contact activator), Russel's viper venom (RVV), and thromboplastin (for dPT). Contact activators that can be used in the methods of the invention as procoagulant reagents include micronized silica particles, ellagic acid, sulfatides, kaolin or the like known to those of skill in the art. Procoagulants may be from a crude natural extract, a blood or plasma sample, isolated and substantially purified, synthetic, or recombinant. Procoagulants may include naturally occurring coagulation factors or fragments, variants or covalently modified derivatives thereof that retain biological activity (*i.e*., promote clotting). Optimal concentrations and dosages of the procoagulant to treat a selected disease can be determined by those of skill in the art.

The term "polysaccharide", as used herein, refers to a polymer comprising a plurality (*i.e*., two or more) of covalently linked saccharide residues. Linkages may be natural or unnatural. Natural linkages include, for example, glycosidic bonds, while unnatural linkages may include, for example, ester, amide, or oxime linking moieties. Polymers may have any of a wide range of average molecular weight (MW) values, but generally are of at least about 100 daltons. For example, the polymers can have molecular weights of at least about 500, 1000, 2000, 4000, 6000, 8000, 10,000, 20,000, 30,000, 50,000, 100,000, 500,000 daltons or even higher. Polymers may have linear chain or branched structures. Polymers may include fragments of polymers generated by degradation (*e.g*., hydrolysis) of larger polymers. Degradation can be achieved by any convenient protocol including treatment of polymers with acid, base, heat, oxidants or enzymes to yield fragmented polymers. Polymers may be chemically altered and may be modified, including but not limited to, sulfation, polysulfation, esterification, and methylation.

In an exemplary embodiment, the NASSP of the invention is not a polysaccharide. Exemplary NASSPs include a saccharide subunit. In various embodiments, the NASSP of the invention is based upon a poly(vinyl) scaffold having one or more sulfate or sulfonate moiety covalently attached directly to the scaffold or to a group pendent from the scaffold, e.g, an aryl or heteroaryl moiety. In various embodiments, the scaffold is a poly(amino acid) scaffold in which the monomers are covalently attached through peptide bonds. The sulfate or sulfonate moiety is covalently attached directly to the scaffold or to a moiety pendent from the scaffold, *e.g*., an aryl or heteroaryl moiety.

"Molecular weight", as discussed herein, can be expressed as either a number average molecular weight or a weight average molecular weight. Unless otherwise indicated, all references to molecular weight herein refer to the weight average molecular weight. Both molecular weight determinations, number average and weight average, can be measured using for example, gel permeation chromatography or other liquid chromatography techniques.

"Pharmaceutically acceptable" means that which is useful in preparing a pharmaceutical composition that is generally safe, non-toxic and neither biologically nor otherwise undesirable and includes that which is acceptable for veterinary use as well as human pharmaceutical use.

The phrase "therapeutically effective amount" as used herein means that amount of a compound, material, or composition comprising a compound of the present invention which is effective for producing a desired therapeutic effect, at a reasonable benefit/risk ratio, such as those generally applicable to the treatment of the bleeding disorder using art-standard pharmaceuticals. "Therapeutically effective dose or amount" of a NASSP, blood factor, or other therapeutic agent refers to an amount of this substance that, when administered as described herein, brings about a positive therapeutic response, such as reduced bleeding or shorter clotting times.

The term "pharmaceutically acceptable salts" is generally relevant to salts such as -SO₃-M⁺ and -OSO₃-M⁺, and includes salts of the active compounds which are prepared with relatively non-toxic acids or bases, depending on the particular substituents found on the compounds described herein. When compounds of the present invention contain relatively acidic functionalities, base addition salts can be obtained by contacting the neutral form of such compounds with a sufficient amount of the desired base, either neat or in a suitable inert solvent. Examples of pharmaceutically acceptable base addition salts include sodium, potassium, calcium, ammonium, organic amino, or magnesium salt, or a similar salt. When compounds of the present invention contain relatively basic functionalities, acid addition salts can be obtained by contacting the neutral form of such compounds with a sufficient amount of the desired acid, either neat or in a suitable inert solvent. Examples of pharmaceutically acceptable acid addition salts include those derived from inorganic acids like hydrochloric, hydrobromic, nitric, carbonic, monohydrogencarbonic, phosphoric, monohydrogenphosphoric, dihydrogenphosphoric, sulfuric, monohydrogensulfuric, hydriodic, or phosphorous acids and the like, as well as the salts derived from relatively non-toxic organic acids like acetic, propionic, isobutyric, maleic, malonic, benzoic, succinic, suberic, fumaric, lactic, mandelic, phthalic, benzenesulfonic, p-tolylsulfonic, citric, tartaric, methanesulfonic, and the like. Also included are salts of amino acids such as arginate and the like, and salts of organic acids like glucuronic or galactunoric acids and the like (see, for example, Berge et al., Journal of Pharmaceutical Science, 66: 1-19 (1977)). Certain specific compounds of the present invention contain both basic and acidic functionalities that allow the compounds to be converted into either base or acid addition salts.

When a residue is defined as "SO₃-", then the formula is meant to optionally include an organic or inorganic cationic counterion. Preferably, the resulting salt form of the compound is pharmaceutically acceptable. This structure also encompasses the protonated species, "SO₃H".

The neutral forms of the compounds are preferably regenerated by contacting the salt with a base or acid and isolating the parent compound in the conventional manner. The parent form of the compound differs from the various salt forms in certain physical properties, such as solubility in polar solvents, but otherwise the salts are equivalent to the parent form of the compound for the purposes of the present invention.

The term "bleeding disorder" as used herein refers to any disorder associated with excessive bleeding, such as a congenital coagulation disorder, an acquired coagulation disorder, or a trauma induced hemorrhagic condition. Such bleeding disorders include, but are not limited to, hem A, hem B, von Willebrand disease, idiopathic thrombocytopenia, a deficiency of one or more coagulation factors, such as Factor XI, Factor XII, prekallikrein, and HMWK, a deficiency of one or more factors associated with clinically significant bleeding, such as Factor V, Factor VII, Factor VIII, Factor IX, Factor X, Factor XIII, Factor II (hypoprothrombinemia), and von Willebrand Factor, a vitamin K deficiency, a disorder of fibrinogen, including afibrinogenemia, hypofibrinogenemia, and dysfibrinogenemia, an alpha2-antiplasmin deficiency, and excessive bleeding such as caused by liver disease, renal disease, thrombocytopenia, platelet dysfunction, hematomas, internal hemorrhage, hemarthroses, surgery, trauma, hypothermia, menstruation, and pregnancy.

By "subject" is meant any member of the subphylum chordata, including, without limitation, humans and other primates, including non human primates such as chimpanzees and other apes and monkey species; farm animals such as cattle, sheep, pigs, goats and horses; domestic mammals such as dogs and cats; laboratory animals including rodents such as mice, rats and guinea pigs; birds, including domestic, wild and game birds such as chickens, turkeys and other gallinaceous birds, ducks, geese, and the like. The term does not denote a particular age. Thus, both adult and newborn individuals are of interest.

The term "patient," is used in its conventional sense to refer to a living organism suffering from or prone to a condition that can be prevented or treated by administration of a NASSP of the invention, and includes both humans and non-human species.

"TFPI" and "flTFPI", as used herein, refer to tissue factor pathway inhibitor and full length tissue factor pathway inhibitor, respectively.

"TFPI160" refers to a polypeptide including amino acid 1-160, including KD1 and KD2 domains, of TFPI. The KD3 and C-terminus of full length TFPI are absent.

### THE EMBODIMENTS

Various aspects of the invention include methods for enhancing blood coagulation in a subject. In practicing methods according to certain embodiments, an amount of a non-anticoagulant sulfated or sulfonated synthetic polymer (NASSP) is administered to a subject in a manner sufficient to enhance blood coagulation in the subject. Compositions and kits for practicing methods of the invention are also provided.

Before the invention is described in greater detail, it is to be understood that the invention is not limited to particular embodiments described herein as such embodiments may vary. It is also to be understood that the terminology used herein is for the purpose of describing particular embodiments only, and the terminology is not intended to be limiting. The scope of the invention will be limited only by the appended claims. Unless defined otherwise, all technical and scientific terms used herein have the same meaning as commonly understood by one of ordinary skill in the art to which this invention belongs. Where a range of values is provided, it is understood that each intervening value, to the tenth of the unit of the lower limit unless the context clearly dictates otherwise, between the upper and lower limit of that range and any other stated or intervening value in that stated range, is encompassed within the invention. The upper and lower limits of these smaller ranges may independently be included in the smaller ranges and are also encompassed within the invention, subject to any specifically excluded limit in the stated range. Where the stated range includes one or both of the limits, ranges excluding either or both of those included limits are also included in the invention. Certain ranges are presented herein with numerical values being preceded by the term "about." The term "about" is used herein to provide literal support for the exact number that it precedes, as well as a number that is near to or approximately the number that the term precedes. In determining whether a number is near to or approximately a specifically recited number, the near or approximating unrecited number may be a number, which, in the context in which it is presented, provides the substantial equivalent of the specifically recited number. All publications, patents, and patent applications cited in this specification are incorporated herein by reference to the same extent as if each individual publication, patent, or patent application were specifically and individually indicated to be incorporated by reference. Furthermore, each cited publication, patent, or patent application is incorporated herein by reference to disclose and describe the subject matter in connection with which the publications are cited. The citation of any publication is for its disclosure prior to the filing date and should not be construed as an admission that the invention described herein is not entitled to antedate such publication by virtue of prior invention. Further, the dates of publication provided might be different from the actual publication dates, which may need to be independently confirmed.

It is noted that the claims may be drafted to exclude any optional element. As such, this statement is intended to serve as antecedent basis for use of such exclusive terminology as "solely," "only," and the like in connection with the recitation of claim elements, or use of a "negative" limitation. As will be apparent to those of skill in the art upon reading this disclosure, each of the individual embodiments described and illustrated herein has discrete components and features which may be readily separated from or combined with the features of any of the other several embodiments without departing from the scope or spirit of the invention. Any recited method may be carried out in the order of events recited or in any other order that is logically possible. Although any methods and materials similar or equivalent to those described herein may also be used in the practice or testing of the invention, representative illustrative methods and materials are now described.

### A. NASSPs

In one aspect, the invention provides a sulfated or sulfonated synthetic polymer with the ability to enhance coagulation of mammalian blood *in vivo* or *in vitro.* In various embodiments, the sulfated or sulfonated synthetic polymer has procoagulant activity. In various aspects the procoagulant activity of the polymer of the invention is of sufficient magnitude that is measurable using a standard coagulation assay, e.g. the Thrombin Generation Assay (TGA).

Exemplary sulfated or sulfonated synthetic polymers of the invention provide a subject administered one of these polymers a therapeutically effective procoagulant effect. Exemplary compounds of the invention also exert an anticoagulant effect upon administration to a subject; in various embodiments, the polymers of the invention do not induce a degree of anticoagulant effect sufficient to entirely offset the therapeutically effective procoagulant effect of the polymer. Exemplary NASSPs of the invention are procoagulant at a concentration of from about 0.1µg/mL to about 700 µg/mL of plasma (*e.g*., human plasma), *e.g*., from about 0.3 µg/mL to about 600 µg/mL plasma, e.g, from about 1 µg/mL to about 500 µg/mL plasma, *e.g*., from about 3 µg/mL to about 400 µg/mL, *e.g.,* from about 10 µg/mL to about 300 µg/mL plasma, *e.g*., from about 20 µg/mL to about 200 µg/mL plasma, *e.g.,* from about 30 µg/mL to about 100 µg/mL plasma. Exemplary NASSPs of the invention are substantially non-anticoagulant at the aforementioned concentrations as thrombin generation is above the hemophilia plasma level as measured in a standard assay such as calibrated automatic thrombography (CAT), examples of which are set forth herein. See, *e.g*., Example 1 and **FIG. 4A-E**. Both the procoagulant effect and peak thrombin can be measured by CAT.

Exemplary NASSPs of the invention are sulfated or sulfonated synthetic polymers with procoagulant activity and anticoagulant activity. The anticoagulant properties of potential NASSPs are determined using the activated partial thromboplastin time (aPTT) clotting assay. Exemplary NASSPs of the invention exhibit no more than one-half, preferably no more than one-third, and preferably less than one-tenth, the anticoagulant activity (measured by increase in clotting time) of unfractionated heparin.

In various embodiments, the invention provides a non-saccharide sulfated or sulfonated synthetic polymer having the formula selected from: 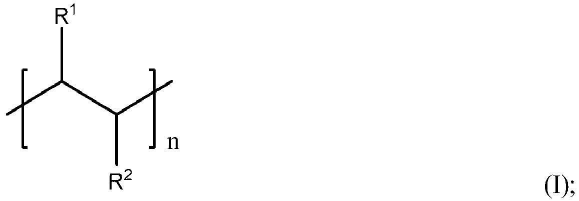 and 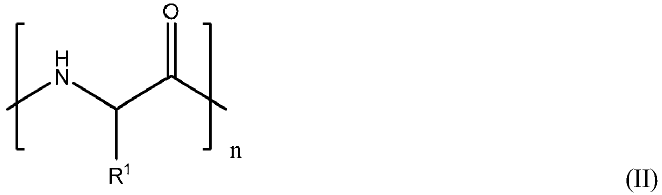 wherein R¹ is selected from H, substituted or unsubsituted alkyl and sulfonated aryl. R² is selected from H, substituted or unsubstituted alkyl and sulfonate, such that at least one of R¹ and R² is or includes a sulfonate or sulfate group (*e.g*., SO₃⁻M⁺, or SO₃H; M⁺ is an inorganic or organic cation). In an exemplary embodiment, only one of R¹ and R² includes a sulfonate or sulfate moiety. The index n represents an integer greater than 0, which signifies the number of repeated subunits in the polymer. In exemplary embodiments, n is selected to provide a polymer with a molecular weight from about 7 kDa to about 300 kDa, for example, from about 9 kDa to about 200 kDa, *e.g*., from about 11 kDa to about 100kDa. In an exemplary embodiment, the value of n is selected such that the polymer has a molecular weight that is not more than about 300 kDa, not more than about 250 kDa, not more than about 200 kDa, not more than 150 kDa, not more than about 100 kDa, not more than about 50 kDa, not more than about 25 kDa or not more than about 10 kDa. In an exemplary embodiment, the value of n is selected such that the polymer has a molecular weight of not more than about 9 kDa, not more than about 8 kDa, not more than about 7 kDa, not more than about 6 kDa, not more than about 5 kDa, not more than about 4 kDa, not more than about 3 kDa, or not more than about 2 kDa.

Exemplary sulfated or sulfonated synthetic polymers of the invention are characterized by providing a subject administered one of these polymers a therapeutically relevant procoagulant effect. Exemplary compounds of the invention also exert an anticoagulant effect upon administration to a subject. In various embodiments, the polymers of the invention do not induce a degree of anticoagulant effect sufficient to entirely offset the procoagulant effect of the polymer. Exemplary NASSPs of the invention are procoagulant at a concentration of from about 0.1µg/mL to about 700 µg/mL of plasma (*e.g*., human plasma), *e.g*., from about 0.3 µg/mL to about 600 µg/mL plasma, e.g, from about 1 µg/mL to about 500 µg/mL plasma, *e.g.,* from about 3 µg/mL to about 400 µg/mL, *e.g*., from about 10 µg/mL to about 300 µg/mL plasma, *e.g*., from about 20 µg/mL to about 200 µg/mL plasma, *e.g*., from about 30 µg/mL to about 100 µg/mL plasma.

In various embodiments, the compounds of the invention have a procoagulant effect at a concentration of not more than about 400 µg/mL, *e.g*., not more than about 350 µg/mL, *e.g*., not more than about 300 µg/mL, *e.g*., not more than about 250 µg/mL, *e.g*., not more than about 200 µg/mL, *e.g*., not more than about 150 µg/mL, *e.g*., not more than about 100 µg/mL, *e.g*., not more than about 50 µg/mL.

In various embodiments, R¹ is: 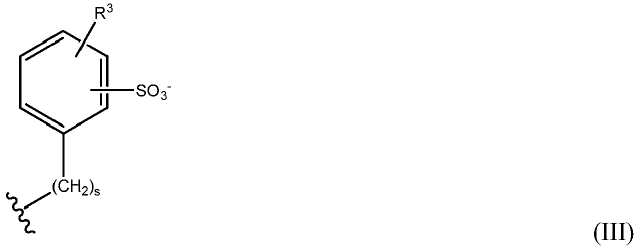 in which R³ is selected from H and OR⁴. The index s is 0, 1, 2, 3 or higher. R⁴ is selected from H, and substituted or unsubstituted alkyl. In various embodiments in which R¹ is the substituted phenyl group (III), R² is H.

Exemplary polymers according to the present invention are set forth in **FIG. 3****.**

In other embodiments the NASSP of the invention decreases blood clotting time when tested in the TFPI- dilute prothrombin time (TFPI-dPT) clotting assay. In various embodiments, a NASSP of the invention reverses the anticoagulant effect of exogenous flTFPI in human plasma. In various embodiments, the compound of the invention interferes with the action of TFPI. In various embodiments, the NASSP of the invention interacts with the C-terminus of TFPI to provide procoagulant activity.

In an exemplary embodiment, the invention provides a sulfated or sulfonated synthetic polymer with procoagulant activity, which is based on polyvinyl, polystyrene, polytyrosine or polyanethole.

In various embodiments, the invention provides NASSPs that include at least about 5%, at least about 10%, at least about 15%, or at least about 20% sulfur. In an exemplary embodiment, this amount of sulfur is determined by elemental analysis of the NASSP.

In an exemplary embodiment, the NASSP is characterized in having procoagulant effects in a standard assay for this property.

In an exemplary embodiment, the procoagulant effect is sufficient for the NASSP to be of use in a method for treating a subject in need of enhanced blood coagulation. The method comprises administering a therapeutically effective amount of a composition comprising a non-anticoagulant, non-saccharide sulfated or sulfonated synthetic polymer (NASSP) to the subject.

In other embodiments the NASSP is selected from one or more low molecular weight fragment of the previously listed compounds. In preferred embodiments the fragment of the NASSP decreases blood clotting time when tested in the TFPI-dPT assay. In one embodiment, the NASSP is a fragment of a sulfate or sulfonate polymer that decreases blood clotting time when tested in the TFPI-dPT assay. In an exemplary embodiment, these fragments have the properties described above with respect to the polymer having a therapeutically effective procoagulant effect even if this effect is offset by its anticoagulant properties.

In further embodiments, the invention provides a NASSP co-formulated with one or more different NASSPs and/or in combination with one or more other therapeutic agents.

The ability of NASSPs to promote clotting and reduce bleeding is readily determined using various *in vitro* clotting assays (*e.g*., TFPI-dPT, thrombin generation and rotational thromboelastometry (ROTEM) assays) and *in vivo* bleeding models (*e.g.* tail transection or cuticle bleeding time determination in hemophilic mice or dogs). See, *e.g*., PDR Staff. Physicians' Desk Reference, 2004, Nordfang, et al. (1991) Thromb Haemost., 66:464-467; Anderson, et al. (1976) Thromb. Res., 9:575-580; Welsch, et al. (1991) Thrombosis Research, 64:213-222; Broze, et al. (2001) Thromb Haemost, 85:747-748; Scallan, et al. (2003) Blood, 102:2031-2037; Pijnappels, et al. (1986) Thromb. Haemost., 55:70-73; and Giles, et al.(1 982) Blood, 60:727-730. Clotting assays may be performed in the presence of NASSPs and one or more blood factors, procoagulants, or other reagents. For example, one or more coagulation factors can be added, including but not limited to, Factor XI, Factor XII, prekallikrein, HMWK, Factor V, Factor VII, Factor VIII, Factor IX, Factor X, Factor XIII, Factor II, and von Willebrand Factor, tissue factor, Factor VIIa, Factor Va, and Factor Xa, Factor IXa, Factor XIa, Factor XIIa, and Factor VIIIa; and/or one or more reagents, including but not limited to, APTT reagent, thromboplastin, fibrin, TFPI, Russell's viper venom, micronized silica particles, ellagic acid, sulfatides, and kaolin.

The Examples and the Figures appended hereto confirm that the agents referred to herein as NASSPs are truly "non-anticoagulant," *i.e*., they do not significantly increase clotting times within a selected concentration range. Such compounds can be used in the methods and compositions of the present invention provided that any anticoagulant activity that they may exhibit only appears at concentrations significantly above the concentration at which they exhibit procoagulant activity. The ratio of the concentration at which undesired anticoagulant properties occur to the concentration at which desired procoagulant activities occur is referred to as the procoagulant index (or, *e.g*., therapeutic index) for the NASSP in question. The procoagulant index for NASSPs of the present invention may be about 5, 10, 30, 100, 300, 1000 or more.

### B. Pharmaceutical Compositions

In various embodiments, the NASSP of the invention is incorporated into a pharmaceutical formulation. In various embodiments, depending on the desired effects and potency of the NASSPs, one or more NASSPs may be formulated together. For example, two or more NASSPs may be formulated together, such as three or more NASSPs and including four or more NASSPs. Where more than one NASSP is employed, the mass percentage of each NASSP in the composition may vary, ranging from 1% or more of the total mass of the composition, such as 2% or more, such as 5% or more, such as 10% or more, such as 25% or more and including as 50% or more of the total mass of the composition.

In various embodiments, the pharmaceutical formulations of the invention optionally contain one or more pharmaceutically acceptable excipient. Exemplary excipients include, without limitation, carbohydrates, inorganic salts, antimicrobial agents, antioxidants, surfactants, buffers, acids, bases, and combinations thereof. Liquid excipients include water, alcohols, polyols, glycerine, vegetable oils, phospholipids, and surfactants. A carbohydrate such as a sugar, a derivatized sugar such as an alditol, aldonic acid, an esterified sugar, and/or a sugar polymer may be present as an excipient. Specific carbohydrate excipients include, for example: monosaccharides, such as fructose, maltose, galactose, glucose, D-mannose, sorbose, and the like; disaccharides, such as lactose, sucrose, trehalose, cellobiose, and the like; polymers, such as raffinose, melezitose, maltodextrins, dextrans, starches, and the like; and alditols, such as mannitol, xylitol, maltitol, lactitol, xylitol, sorbitol (glucitol), pyranosyl sorbitol, myoinositol, and the like. The excipient can also include an inorganic salt or buffer such as citric acid, sodium chloride, potassium chloride, sodium sulfate, potassium nitrate, sodium phosphate monobasic, sodium phosphate dibasic, and combinations thereof.

A composition of the invention can also include an antimicrobial agent for preventing or deterring microbial growth. Non-limiting examples of antimicrobial agents suitable for the present invention include benzalkonium chloride, benzethonium chloride, benzyl alcohol, cetylpyridinium chloride, chlorobutanol, phenol, phenylethyl alcohol, phenylmercuric nitrate, thimersol, and combinations thereof.

An antioxidant can be present in the composition as well. Antioxidants are used to prevent oxidation, thereby preventing the deterioration of the NASSP or other components of the preparation. Suitable antioxidants for use in the present invention include, for example, ascorbyl palmitate, butylated hydroxyanisole, butylated hydroxytoluene, hypophosphorous acid, monothioglycerol, propyl gallate, sodium bisulfite, sodium formaldehyde sulfoxylate, sodium metabisulfite, and combinations thereof.

A surfactant can be present as an excipient. Exemplary surfactants include: polysorbates, such as "Tween 20" and "Tween 80", and pluronics such as F68 and F88 (BASF, Mount Olive, N.J.); sorbitan esters; lipids, such as phospholipids such as lecithin and other phosphatidylcholines, phosphatidylethanolamines (although preferably not in liposomal form), fatty acids and fatty esters; steroids, such as cholesterol; chelating agents, such as EDTA; and zinc and other such suitable cations.

Acids or bases can be present as an excipient in the composition. Non-limiting examples of acids that can be used include those acids selected from the group consisting of hydrochloric acid, acetic acid, phosphoric acid, citric acid, malic acid, lactic acid, formic acid, trichloroacetic acid, nitric acid, perchloric acid, phosphoric acid, sulfuric acid, fumaric acid, and combinations thereof. Examples of suitable bases include, without limitation, bases selected from the group consisting of sodium hydroxide, sodium acetate, ammonium hydroxide, potassium hydroxide, ammonium acetate, potassium acetate, sodium phosphate, potassium phosphate, sodium citrate, sodium formate, sodium sulfate, potassium sulfate, potassium fumnerate, and combinations thereof.

The amount of the NASSP in the composition will vary depending on a number of factors, but will optimally be a therapeutically effective dose when the composition is in a unit dosage form (*e.g*., a pill, or capsule) or container (*e.g*., a vial or bag). A therapeutically effective dose can be determined experimentally by repeated administration of increasing amounts of the composition in order to determine which amount produces a clinically desired endpoint.

The amount of any individual excipient in the composition will vary depending on the nature and function of the excipient and particular needs of the composition. Typically, the optimal amount of any individual excipient is determined through routine experimentation, *i.e*., by preparing compositions containing varying amounts of the excipient (ranging from low to high), examining the stability and other parameters, and then determining the range at which optimal performance is attained with no significant adverse effects. Generally, however, the excipient(s) is present in the composition in an amount of about 1% to about 99% by weight, preferably from about 5% to about 98% by weight, more preferably from about 15% to about 95% by weight of the excipient, with concentrations less than 30% by weight most preferred. These foregoing pharmaceutical excipients along with other excipients are described in "Remington: The Science & Practice of Pharmacy", 19th ed., Williams & Williams, (1995), the "Physician's Desk Reference", 52nd ed., Medical Economics, Montvale, N.J. (1998), and Kibbe, A. H., Handbook of Pharmaceutical Excipients, 3rd Edition, American Pharmaceutical Association, Washington, D.C., 2000.

The compositions encompass all types of formulations and in particular those that are suited for oral administration or injection. Additional preferred compositions include those for oral, ocular, or localized delivery.

The pharmaceutical preparations herein can also be housed in an infusion bag, a syringe, an implantation device, or the like, depending upon the intended mode of delivery and use. Preferably, the NASSP compositions described herein are in unit dosage form, meaning an amount of a conjugate or composition of the invention appropriate for a single dose, in a premeasured or pre-packaged form.

The formulations can conveniently be presented in unit dosage form and can be prepared by any of the methods well known in the art of pharmacy. All methods include the step of bringing into association a compound or a pharmaceutically acceptable salt or solvate thereof ("active ingredient") with the carrier which constitutes one or more accessory ingredients. In general, the formulations are prepared by uniformly and intimately bringing into association the active ingredient with liquid carriers or finely divided solid carriers or both and then, if necessary, shaping the product into the desired formulation. Oral formulations are well known to those skilled in the art, and general methods for preparing them are found in any standard pharmacy school textbook, for example, Remington: The Science and Practice of Pharmacy, A.R. Gennaro, ed. (1995), the entire disclosure of which is incorporated herein by reference.

In various embodiments, the invention provides a unit dosage formulation including one or more NASSP of the invention. In an exemplary embodiment, the unit dosage formulation includes a therapeutically effective dosage of a NASSP, preferably sufficient to invoke a clinically detectable and, preferably, a clinically meaningful alteration in the clotting status of the subject to whom the single dosage formulation is administered. In exemplary embodiments, the amount of a NASSP of the invention in the formulation ranges from and amount sufficient to provide a dosage of about 0.01 mg/kg to about 200 mg/kg of a NASSP. In various embodiments, the amount of a NASSP of the invention is sufficient to provide a dosage of from about 0.01 mg/kg to 20 mg/kg, *e.g*., from about 0.02 mg/kg to 2 mg/kg. The amount of compound in the unit dosage formulation will depend on the potency of the specific NASSP and the magnitude or procoagulant effect desired and the route of administration.

Exemplary unit dosage formulations are those containing an effective dose, or an appropriate fraction thereof, of the active ingredient, or a pharmaceutically acceptable salt thereof. A prophylactic or therapeutic dose typically varies with the nature and severity of the condition to be treated and the route of administration. The dosage, and perhaps the dosing frequency, will also vary according to the age, body weight and response of the individual patient. In general, for the compounds of the invention, the total dose in a unit dosage form of the invention ranges from about 1 mg to about 7000 mg, *e.g*., from about 2 mg to about 500 mg, *e.g*., from about 10 mg to about 200 mg, *e.g*., from about 20 mg to about 100 mg, *e.g*., from about 20 mg to about 80 mg, *e.g*., from about 20 mg to about 60 mg. In some embodiments, the amount of a NASSP of the invention in a unit dosage form ranges from about 50 mg to about 500 mg, *e.g*., from about 100 mg to about 200 mg.

The NASSP formulations herein may optionally include one or more additional agents, such as hemostatic agents, blood factors, or other medications used to treat a subject for a condition or disease. In various embodiments, the invention provides combination preparations including one or more blood factors such as Factor XI, Factor XII, prekallikrein, HMWK, Factor V, Factor VII, Factor VIII, Factor IX, Factor X, Factor XIII, Factor II, Factor VIIa, and von Willebrand Factor, prothrombin complex concentrate (PCC), activated prothrombin complex concentrate (aPCC), *e.g*., FEIBA. NASSP compositions may also include other procoagulants, such as an activator of the intrinsic coagulation pathway, including but not limited to, Factor Xa, Factor IXa, Factor XIa, Factor XIIa, and VIIIa, prekallikrein, and HMWK; or and activator of the extrinsic coagulation pathway, including but not limited to, tissue factor, Factor VIIa, Factor Va, and Factor Xa. NASSP compositions may include naturally occurring, synthetic, or recombinant coagulation factors or fragments, variants or covalently modified derivatives thereof that retain biological activity (*i.e*., promote clotting). Alternatively, such agents can be contained in a separate composition from the NASSP and co-administered concurrently, before, or after the NASSP composition of the invention.

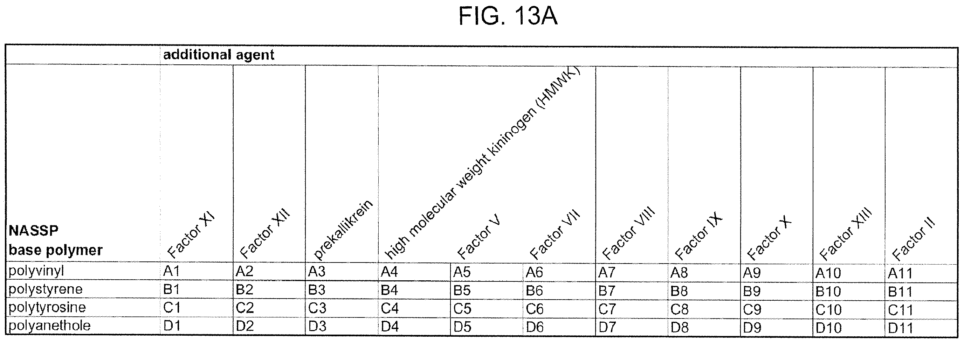

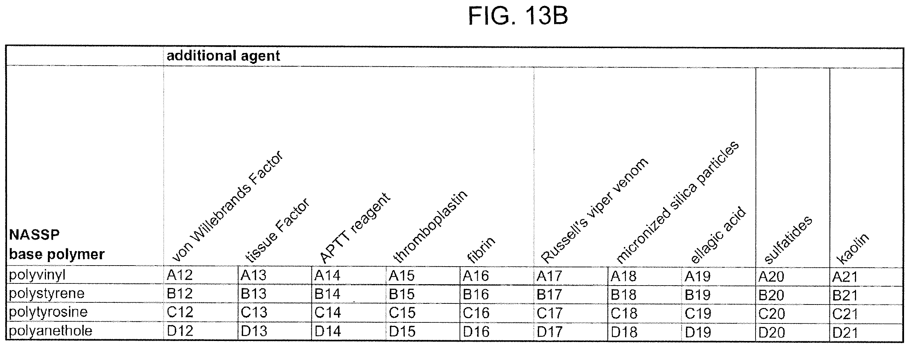

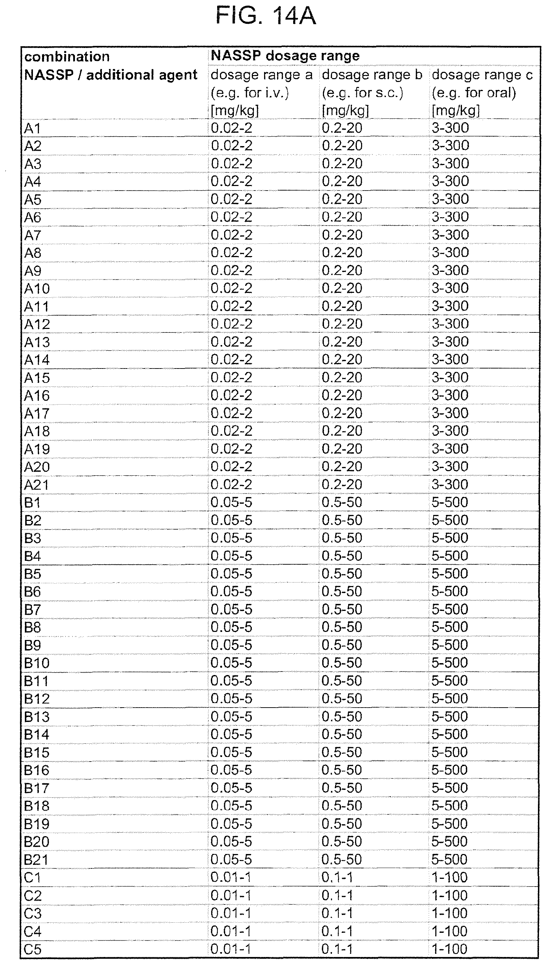

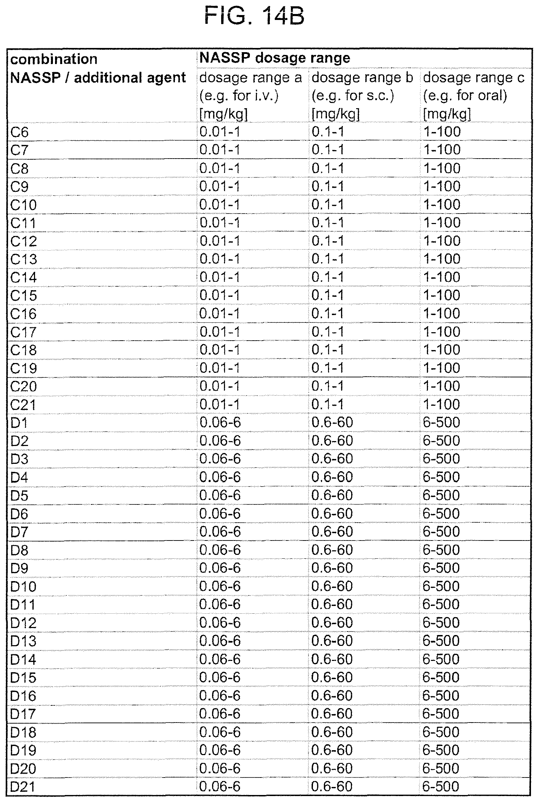

Exemplary combinations of certain types of NASSPs (based on their base polymer) with additional agents are shown in **FIG. 13A-B****.** Each combination therein is identified by a capital letter (referring to a type of NASSP) followed by a number (referring to the additional agent). For example, "C5" refers to the combination of a NASSP having a polytyrosine base polymer with Factor V. **FIG. 14A-B** provides exemplary dosage ranges for the respective NASSP in each of the combinations (type of NASSP (based on the base polymer) with additional agent) identified in **FIG. 13A-B****.**

The individual components of such combinations may be administered either simultaneously or sequentially in a unit dosage form. The unit dosage form may be a single or multiple unit dosage form. In an exemplary embodiment, the invention provides a combination in a single unit dosage form. An example of a single unit dosage form is a capsule wherein both the compound of the invention and the additional therapeutic agent are contained within the same capsule. In an exemplary embodiment, the invention provides a combination in a two unit dosage form. An example of a two unit dosage form is a first capsule which contains the compound of the invention and a second capsule which contains the additional therapeutic agent. Thus the term 'single unit' or 'two unit' or 'multiple unit' refers to the object which the patient ingests, not to the interior components of the object. Appropriate doses of known therapeutic agents will be readily appreciated by those skilled in the art.

In various embodiments, the NASSP compositions herein may, when intended for oral administration, optionally include one or more permeation enhancer. Appropriate permeation enhancers and their use with procoagulants, such as those provided by the present invention are disclosed in U.S. Provisional Patent Application No. 61/509,514, filed July 19, 2011, titled "Absorption Enhancers as Additives to Improve the Oral Formulation of Non-Anticoagulant Sulfated Polysaccharides". In some embodiments the permeation enhancer is a gastrointestinal epithelial barrier permeation enhancer. Depending on the physiology of the subject, the phrase "gastrointestinal epithelial" as used herein, refers to the epithelial tissue of the digestive tract, such as the stomach and intestinal tract (*e.g*., duodenum, jejunum, ileum), and may further include other structures which participate in the gastrointestinal functions of the body including the lower part of the esophagus, the rectum and the anus. In various embodiments, compositions of the invention include a procoagulant amount of a NASSP in combination with a gastrointestinal epithelial barrier permeation enhancer. Amounts of permeation enhancer of use in this invention are generally identical to those set forth in above-referenced U.S. Provisional Patent Application No. 61/509,514. Similarly, in exemplary embodiments, appropriate amounts of an NASSP are identical to those amounts set forth for NASPs in the above-referenced application. In various embodiments, compositions of the invention include a combination of a procoagulant amount of a NASSP with a gastrointestinal epithelial barrier permeation enhancer and a blood coagulation factor. Exemplary amounts of NASSP and a second agent, e.g, a blood coagulation factor, are set forth herein. Gastrointestinal epithelial barrier permeation enhancers include compounds that, when orally administered, increase the amount of NASSP that is absorbed by the gastrointestinal system. Furthermore, gastrointestinal permeation enhancers may also accelerate the initiation (*i.e*., reducing the amount time for absorption to begin) of NASSP absorption through the gastrointestinal epithelium as well as accelerate the overall rate of transport of the NASSP across the gastrointestinal epithelium of the subject (*i.e*., reducing the amount of time for NASSP absorption by the gastrointestinal system to be complete). In embodiments of the invention, gastrointestinal epithelial barrier permeation enhancers may vary, depending on the particular blood coagulation disorder, the physiology of the subject and the desired enhancement of absorption by the gastrointestinal system. In some embodiments, gastrointestinal epithelial barrier permeation enhancers are tight junction modulators. The term "tight junction" is employed in its conventional sense to refer to the closely associated cellular areas where membranes of adjacent cells are joined together. In embodiments of the invention, tight junction modulators may include, but are not limited to enzymes, bile acids, polysaccharides, fatty acids and salts thereof and any combination thereof.

In an exemplary embodiment of the invention the invention provides a pharmaceutical formulation comprising a) a compound of the invention; b) an additional therapeutic agent and c) a pharmaceutically acceptable excipient. In an exemplary embodiment, the pharmaceutical formulation is a unit dosage form. In an exemplary embodiment, the pharmaceutical formulation is a single unit dosage form. In an exemplary embodiment, the pharmaceutical formulation is a two unit dosage form. In an exemplary embodiment, the pharmaceutical formulation is a two unit dosage form comprising a first unit dosage form and a second unit dosage form, wherein the first unit dosage form includes a) a compound of the invention and b) a first pharmaceutically acceptable excipient; and the second unit dosage form includes c) an additional therapeutic agent and d) a second pharmaceutically acceptable excipient.

It should be understood that in addition to the ingredients particularly mentioned above, the formulations of this invention can include other agents conventional in the art having regard to the type of formulation in question, for example those suitable for oral administration can include flavoring agents.

Formulations of the present invention suitable for oral administration can be presented as discrete units such as capsules (*e.g*., soft-gel capsules), cachets or tablets each containing a predetermined amount of the active ingredient; as a powder or granules; as a solution or a suspension in an aqueous liquid or a non-aqueous liquid; or as an oil-in-water liquid emulsion or a water-in-oil liquid emulsion. The active ingredient can also be presented as a bolus, electuary or paste.

A tablet can be made by compression or molding, optionally using one or more accessory ingredients. Compressed tablets can be prepared by compressing in a suitable machine the active ingredient in a free-flowing form such as a powder or granules, optionally mixed with a binder, lubricant, inert diluent, lubricating, surface active or dispersing agent. Molded tablets can be made by molding in a suitable machine a mixture of the powdered compound moistened with an inert liquid diluent. The tablets can optionally be coated or scored and can be formulated so as to provide sustained, delayed or controlled release of the active ingredient therein. Oral and parenteral sustained release drug delivery systems are well known to those skilled in the art, and general methods of achieving sustained release of orally or parenterally administered drugs are found, for example, in Remington: The Science and Practice of Pharmacy, pages 1660-1675 (1995), the disclosure of which is incorporated herein by reference.

In an exemplary embodiment, the invention provides a unit dosage formulation of a NASSP of the invention in a form appropriate for administration by injection (*e.g*., infusion). The unit dosage formulation can be diluted with an appropriate pharmaceutically acceptable diluent shortly prior to use, or it can be packaged as a diluted unit dosage for infusion. Suitable forms for dilution prior to injection include, *e.g*., powders or lyophilates that can be reconstituted with a solvent prior to use, as well as ready for injection solutions or suspensions, dry insoluble compositions for combination with a vehicle prior to use, and emulsions and liquid concentrates for dilution prior to administration. Examples of suitable diluents for reconstituting solid compositions prior to injection include bacteriostatic water for injection, dextrose 5% in water, phosphate buffered saline, Ringer's solution, saline, sterile water, deionized water, and combinations thereof. With respect to liquid pharmaceutical compositions, solutions and suspensions are envisioned. In general, the amounts discussed above as appropriate for oral unit dosage forms are applicable to the injectable unit dosage as well.

In a further exemplary embodiment, the invention provides the injectable unit dosage formulation and a device for administration of the unit dosage formulation by injection (*e.g*., infusion). In various embodiments, the device is a device for infusion, *e.g*., an infusion bag. In an example of this embodiment, the invention provides an infusion bag, or similar device, into which the unit dosage formulation is pre-charged (diluted or in a form, appropriate for dilution).

Sulfated or sulfonated synthetic polymers with NASSP activity (*i.e*., procoagulant activity) usefully incorporated into formulations of the invention include, but are not limited to, sulfated or sulfonated synthetic polymers in which the base polymer is selected from polyvinyl, polyanethole, polytyrosine and polystyrene.

### C. NASSPs as Promoters of Clotting

The present invention is based on the discovery that non-anticoagulant sulfated or sulfonated synthetic polymers (NASSPs) can be used as procoagulants in treatment of patients with bleeding disorders. A novel approach for regulating hemostasis has been discovered by the inventors that utilizes sulfated or sulfonated synthetic polymers, (*i.e*., heparin-like) to promote clotting. Selected sulfated or sulfonated synthetic polymers described herein are largely devoid of anticoagulant activity, or exhibit clot-promoting activity at concentrations significantly lower than the concentration at which they exhibit anticoagulant activity, and are hence denoted "non-anticoagulant sulfated or sulfonated synthetic polymers."

As shown in Example 1, NASSPs promote clotting of plasma from subjects that have hemophilia A (hem A) or hemophilia B (hem B) according to thrombin generation and TFPI-dPT clotting assays. In the experiments disclosed herein, certain candidate NASSPs are shown in clotting assays to demonstrate at least ten-fold lower anticoagulant activity as compared to heparin. These results indicate that systemic administration of select NASSPs represents a unique approach for regulating hemostasis in bleeding disorders. Thus, in various embodiments the invention relates to the use of NASSPs to control hemostasis in subjects with bleeding disorders, including congenital coagulation disorders, acquired coagulation disorders, and trauma induced hemorrhagic conditions.

In exemplary embodiments, the NASSPs of the invention have an EC50, as measured in a CAT assay of from about 0.001 µg/mL to about 30 µg/mL of plasma, *e.g*., from about 0.01 µg/mL to about 10 µg/mL, *e.g*., from about 0.05 µg/mL to about 5 µg/mL. In various embodiments, the NASSP of the invention is not substantially anticoagulant at its EC50. An exemplary NASSP of the invention is not substantially anticoagulant at a concentration of up to about 1.1x, 1.3x. 1.6x. 1.9x, 2.5x, 3x, 3.5x, 4.0x its EC₅₀.

### D. Applications

In one aspect, NASSPs may be used in the methods of the invention for improving hemostasis in treating bleeding disorders, particularly those associated with deficiencies of coagulation factors or for reversing the effects of anticoagulants in a subject. NASSPs may be administered to a subject to treat bleeding disorders, including congenital coagulation disorders, acquired coagulation disorders, and hemorrhagic conditions induced by trauma. Examples of bleeding disorders that may be treated with NASSPs include, but are not limited to, hem A, hem B, von Willebrand disease, idiopathic thrombocytopenia, a deficiency of one or more coagulation factors, such as Factor XI, Factor XII, prekallikrein, and HMWK, a deficiency of one or more factors associated with clinically significant bleeding, such as Factor V, Factor VII, Factor VIII, Factor IX, Factor X, Factor XIII, Factor II (hypoprothrombinemia), and von Willebrand Factor, a vitamin K deficiency, a disorder of fibrinogen, including afibrinogenemia, hypofibrinogenemia, and dysfibrinogenemia, an alpha2-antiplasmin deficiency, and excessive bleeding such as caused by liver disease, renal disease, thrombocytopenia, platelet dysfunction, hematomas, internal hemorrhage, hemarthroses, surgery, trauma, hypothermia, menstruation, and pregnancy. In certain embodiments, NASSPs are used to treat congenital coagulation disorders including hem A, hem B, and von Willebrand disease. In other embodiments, NASSPs are used to treat acquired coagulation disorders, including deficiencies of Factor VIII, von Willebrand Factor, Factor IX, Factor V, Factor XI, Factor XII and Factor XIII, particularly disorders caused by inhibitors or autoimmunity against blood coagulation factors, or haemostatic disorders caused by a disease or condition that results in reduced synthesis of coagulation factors.

### E. Administration

In an exemplary embodiment, at least one therapeutically effective cycle of treatment with a NASSP is administered to a subject. By "therapeutically effective cycle of treatment" is intended a cycle of treatment that when administered, brings about a positive therapeutic response with respect to treatment of an individual for a bleeding disorder. Of particular interest is a cycle of treatment with a NASSP that improves hemostasis. For example, one or more therapeutically effective cycles of treatment may increase the rate of clotting as determined by global hemostatic and clotting assays (*e.g*., CAT, aPTT, described in detail below) by 1% or more, such as 5% or more, such as 10% or more, such as 15% or more, such as 20% or more, such as 30% or more, such as 40% or more, such as 50% or more, such as 75% or more, such as 90% or more, such as 95% or more, including increasing thrombin generation by 99% or more. In other instances, one or more therapeutically effective cycles of treatment may increase clot formation by 1.5-fold or more, such as 2-fold or more, such as 5-fold or more, such as 10-fold or more, such as 50-fold or more, including increasing the formation by 100-fold or more. In some embodiments, subjects treated by methods of the invention exhibit a positive therapeutic response. As used herein, "positive therapeutic response" means that the individual undergoing treatment according to the invention exhibits an improvement in one or more symptoms of a bleeding disorder, including such improvements as shortened blood clotting times and reduced bleeding and/or reduced need for factor replacement therapy.

The invention also provides a method for administering a conjugate comprising a NASSP as provided herein to a patient suffering from a condition that is responsive to treatment with a NASSP contained in the conjugate or composition. The method comprises administering, via any of the herein described modes, a therapeutically effective amount of the conjugate or drug delivery system, preferably provided as part of a pharmaceutical composition. The method of administering may be used to treat any condition that is responsive to treatment with a NASSP. More specifically, the compositions herein are effective in treating bleeding disorders, including hem A, hem B, von Willebrand disease, idiopathic thrombocytopenia, a deficiency of one or more coagulation factors, such as Factor XI, Factor XII, prekallikrein, and HMWK, a deficiency of one or more factors associated with clinically significant bleeding, such as Factor V, Factor VII, Factor VIII, Factor IX, Factor X, Factor XIII, Factor II (hypoprothrombinemia), and von Willebrand Factor, a vitamin K deficiency, a disorder of fibrinogen, including afibrinogenemia, hypofibrinogenemia, and dysfibrinogenemia, an alpha2-antiplasmin deficiency, and excessive bleeding such as caused by liver disease, renal disease, thrombocytopenia, platelet dysfunction, hematomas, internal hemorrhage, hemarthroses, surgery, trauma, hypothermia, menstruation, and pregnancy.

In certain embodiments, one or more therapeutically effective doses of compositions comprising one or more NASSPs and/or other therapeutic agents, such as hemostatic agents, blood factors, or other medications are administered. The compositions of the present invention are typically, although not necessarily, administered orally, via injection (subcutaneously, intravenously or intramuscularly), by infusion, or locally. The pharmaceutical preparation can be in the form of a liquid solution or suspension immediately prior to administration, but may also take another form such as a syrup, cream, ointment, tablet, capsule, powder, gel, matrix, suppository, or the like. Additional modes of administration are also contemplated, such as pulmonary, rectal, transdermal, transmucosal, intrathecal, pericardial, intraarterial, intracerebral, intraocular, intraperitoneal, and so forth. The pharmaceutical compositions comprising NASSPs and other agents may be administered using the same or different routes of administration in accordance with any medically acceptable method known in the art.

A NASSP can be administered prior to, concurrent with, or subsequent to other agents. If provided at the same time as other agents, the NASSP can be provided in the same or in a different composition. Thus, NASSPs and other agents can be presented to the individual by way of concurrent therapy. By "concurrent therapy" is intended administration to a subject such that the therapeutic effect of the combination of the substances is caused in the subject undergoing therapy. For example, concurrent therapy may be achieved by administering a dose of a pharmaceutical composition comprising a NASSP and a dose of a pharmaceutical composition comprising at least one other agent, such as a hemostatic agent or coagulation factor, including, for example, one or more blood factors such as Factor XI, Factor XII, prekallikrein, HMWK, Factor V, Factor VII, Factor VIII, Factor IX, Factor X, Factor XIII, Factor II, Factor VIIa, and von Willebrand Factor. NASSP compositions may also include other procoagulants, such as an activator of the intrinsic coagulation pathway, including but not limited to, Factor Xa, Factor IXa, Factor XIa, Factor XIIa, and VIIIa, prekallikrein, and HMWK; or and activator of the extrinsic coagulation pathway, including but not limited to, tissue factor, Factor VIIa, Factor Va, and Factor Xa, which in combination comprise a therapeutically effective dose, according to a particular dosing regimen. Similarly, one or more NASSPs and therapeutic agents can be administered in at least one therapeutic dose. When the NASSPs and other therapeutic agent(s) are administered as separate pharmaceutical compositions, administration of the separate pharmaceutical compositions can be performed simultaneously or at different times (*i.e*., sequentially, in either order, on the same day, or on different days), so long as the therapeutic effect of the combination of these substances is caused in the subject undergoing therapy.

In a particular embodiment, a composition of the invention is used for localized delivery of a NASSP, for example, for the treatment of bleeding as a result of a lesion, injury, or surgery. The preparations according to the invention are also suitable for local treatment. For example, a NASSP may be administered by injection at the site of bleeding or in the form of a solid, liquid, or ointment, preferably via an adhesive tape or a wound cover. Suppositories, capsules, in particular gastric-juice-resistant capsules, drops or sprays may also be used. The particular preparation and appropriate method of administration are chosen to target the site of bleeding.

In another embodiment, the pharmaceutical compositions comprising NASSPs and/or other agents are administered prophylactically, *e.g*., before a planned surgery. Though of general utility, such prophylactic uses are of particular value for subjects with known pre-existing blood coagulation disorders.

In another embodiment of the invention, the pharmaceutical compositions comprising NASSPs and/or other agents, are in a sustained-release formulation, or a formulation that is administered using a sustained-release device. Such devices are well known in the art, and include, for example, transdermal patches, and miniature implantable pumps that can provide for drug delivery over time in a continuous, steady-state fashion at a variety of doses to achieve a sustained-release effect with a non-sustained-release pharmaceutical composition.

A prophylactic or therapeutic dose typically varies with the nature and severity of the condition to be treated and the route of administration. The dosage, and perhaps the dosing frequency, will also vary according to the age, body weight and response of the individual patient. In general, the total daily dose (in single or divided doses) ranges from about 1 mg per day to about 7000 mg per day, *e.g*., about 1 mg per day to about 100 mg per day, *e.g*., from about 10 mg per day to about 100 mg per day, *e.g*., from about 20 mg to about 100 mg, *e.g*., about 20 mg to about 80 mg, *e.g*., about 20 mg to about 60 mg. In some embodiments, the total daily dose can range from about 50 mg to about 500 mg per day, *e.g*., about 100 mg to about 500 mg per day. It is further recommended that children (*e.g*., infants), patients over 65 years old, and those with impaired renal or hepatic function, initially receive low doses and that the dosage be titrated based on individual physiological responses and/or pharmacokinetics. It can be necessary to use dosages outside these ranges in some cases, as will be apparent to those in the art. Further, it is noted that the clinician or treating physician knows how and when to interrupt, adjust or terminate therapy in conjunction with an individual patient's response.

Those of ordinary skill in the art will appreciate which conditions a specific NASSP can effectively treat. The actual dose to be administered will vary depending upon the age, weight, and general condition of the subject as well as the severity of the condition being treated, the judgment of the health care professional, and conjugate being administered. Therapeutically effective amounts can be determined by those skilled in the art, and are adjusted to the particular requirements of each particular case.

The invention also provides a method for reversing the effects of an anticoagulant in a subject. The method includes administering a therapeutically effective amount of a composition comprising a NASSP to the subject. In certain embodiments, the subject may have been treated with an anticoagulant including, but not limited to, heparin, a coumarin derivative, such as warfarin or dicumarol, TFPI, AT III, lupus anticoagulant, nematode anticoagulant peptide (NAPc2), active-site blocked Factor VIIa (Factor VIIai), Factor IXa inhibitors, Factor Xa inhibitors, including fondaparinux, idraparinux, DX-9065a, and razaxaban (DPC906), inhibitors of Factors Va and VIIIa, including activated protein C (APC) and soluble thrombomodulin, thrombin inhibitors, including hirudin, bivalirudin, argatroban, and ximelagatran. In certain embodiments, the anticoagulant in the subject may be an antibody that binds a coagulation factor, including but not limited to, an antibody that binds to Factor V, Factor VII, Factor VIII, Factor IX, Factor X, Factor XIII, Factor II, Factor XI, Factor XII, von Willebrand Factor, prekallikrein, or HMWK.

In another aspect, the invention provides a method for improving clotting in a subject undergoing a surgical or invasive procedure, the method comprising administering a therapeutically effective amount of a composition comprising a non-anticoagulant sulfated or sulfonated synthetic polymer (NASSP) to the subject. In certain embodiments, the NASSP can be administered alone or co-administered with one or more different NASSPs and/or in combination with one or more other therapeutic agents to the subject undergoing a surgical or invasive procedure. For example, the subject may be administered a therapeutically effective amount of one or more factors selected from the group consisting of Factor XI, Factor XII, prekallikrein, HMWK, Factor V, Factor VII, Factor VIII, Factor IX, Factor X, Factor XIII, Factor II, Factor VIIa, and von Willebrand Factor. Treatment may further comprise administering a procoagulant, such as an activator of the intrinsic coagulation pathway, including Factor Xa, Factor IXa, Factor XIa, Factor XIIa, and Factor VIIIa, prekallikrein, and HMWK; or an activator of the extrinsic coagulation pathway, including tissue factor, Factor VIIa, Factor Va, and Factor Xa.

In addition to the uses set forth above, the compounds of the invention find use in a variety of other treatment modalities. For example, in one embodiment, the compounds of the invention are of use to treat interstitial cystitis (see, *e.g*., Urology, (2000, Dec.) 164(6):2119-2125; Urology, (1999, June) 53(6):1133-1139; International Congress Series, (2001, Dec.) 1223:227-237; Urology, (2008, Jan.) 179(1):177-185; European Urology Supplements, (2003, Sept.) 2(4):14-16; Urology, (2011, Sept.) 78(3):S210-S211; European Urology Supplements, (2011, Oct.) 10(6)451-459; Urology, (2011, April) 185(4):e384).

In various embodiments, the compounds of the invention also find use as antiinflammatory agents, and in the treatment and prevention of neurodegenerative disorders (see, e.g., Food and Chemical Toxicology, (2011, Aug.) 49(8):1745-1752; Food and Chemical Toxicology, (2011, Sept.) 49(9):2090-2095; Biochimica et Biophysica Acta (BBA)-Proteins & Proteomics, (2003, Sept.) 1651(1-2)).

In an exemplary embodiment, compounds of the invention also find use for their anti-cancer activity (see, e.g., Carbohydrate Polymers, (2012, Jan. 4) 87(1, 4):186-194; Carbohydrate Polymers, (2010, May 23) 81(1, 23):41-48; Carbohydrate Polymers, (2012, Jan. 4) 87(1, 4):186-194; International Journal of Biological Macromolecules, (2011, Oct. 1) 49(3, 1):331-336; Advances in Food and Nutrition Research, (2011) 64:391-402).

In various embodiments, the compounds of the invention also find use as agents for the prevention of adhesion formation (see, e.g., Journal of Surgical Research, (2011, Dec.) 171(2):495-503; Fertility and Sterility, (2009, Sept.) 92(3):S58; Journal of Minimally Invasive Gynecology, (2009, Nov.-Dec.) 16(6):S120).

In an exemplary embodiment, compounds of the invention also have antiviral activity (see, e.g., Phytomedicine, (1999, Nov.) 6(5):335-340; Antiviral Research, (1991, Feb.) 15(2):139-148; Phytochemistry, (2010, Feb.) 71(2-3):235-242; and Advances in Food and Nutrition Research, (2011) 64:391-402).

In various embodiments, compounds of the invention are also inhibitors of the complement system (see, e.g., Comparative Biochemistry and Physiology Part C: Pharmacology, Toxicology and Endocrinology, (2000, July) 126(3):209-215).

In each of these different treatment modalities, treatment of a subject in need of such treatment is effected by administering to the subject a therapeutically effective amount of an agent of the invention. In various embodiments, the compound is administered to a subject to treat a condition and this subject is not otherwise in need of treatment with a compound of the invention for a different condition.

In practicing methods of the invention, protocols for enhancing blood coagulation in or treating a condition in a subject may vary, such as for example by age, weight, severity of the blood clotting disorder, the general health of the subject, as well as the particular composition and concentration of the NASSPs being administered. In embodiments of the invention, the plasma concentration of NASSPs achieved in a subject following oral administration and absorption by the gastrointestinal system may vary, in some instances, ranging from about 0.01 µg/mL to about 500 µg/mL. Exemplary NASSPs of interest are procoagulant at their optimal concentration. By "optimal concentration" is meant the concentration in which NASSPs exhibit the highest amount of procoagulant activity. Since exemplary NASSPs also demonstrated anticoagulant activity at much higher concentrations than the optimal concentration, preferred NASSPs of the invention show non-anticoagulant behavior in the range of its optimal concentration. As such, depending on the potency of the NASSP as well as the desired effect, the optimal concentration of an exemplary NASSPs provided by methods of the invention may range, from about 0.01 µg/mL to about 500 µg/mL, such as about 0.1 µg/mL to about 250 µg/mL, such as about 0.1 µg/mL to about 100 µg/mL, such as about 0.1 µg/mL to about 75 µg/mL, such as about 0.1 µg/mL to about 50 µg/mL, such as about 0.1 µg/mL to about 25 µg/mL, such as about 0.1 µg/mL to about 10 µg/mL, and including about 0.1 µg/mL to about 1 µg/mL. Optimal concentrations and activity level as determined by CAT assay of NASPs of interest are described in greater detail in United States Patent Application Serial No. 11/140,504, filed on May 27, 2005, now United States Patent No. 7,767,654, and United States Patent Application Serial No. 13/006,396, filed on January 13, 2011, the disclosures of which is herein incorporated by reference in their entirety. Likewise, the present application discloses examples of CAT assays of use in determining optimal concentration of a NASSP of the invention.

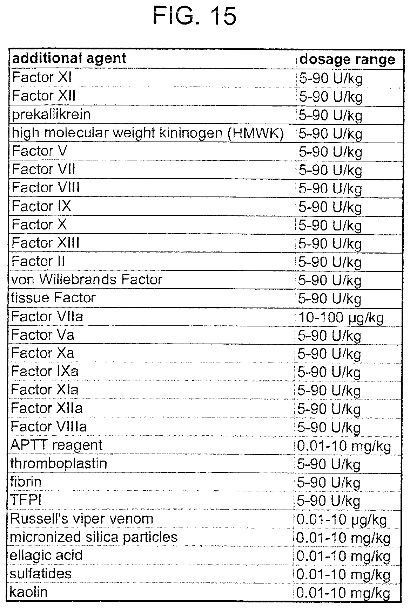

Exemplary dosage ranges for the respective NASSP in each of the combinations (type of NASSP (based on the base polymer) with additional agent) identified in **FIG. 13A-B** are shown in **FIG. 14A-B****.** The lower case letter appended to the identifier of the combination from **FIG. 14A-B** refers to the dosage of the respective NASP in that combination. For example, "C5a" refers to a combination of a NASP having a polytyrosine base polymer with Factor V, wherein the dose of the NASSP is from about 0.01 to about 1 mg/kg. Exemplary dosages for the additional agent are provided in **FIG. 15****.**

In the various embodiments of the invention, the dosage (*e.g*., oral dosage) of compositions containing NASSPs of the invention may vary, in exemplary embodiments, ranging from about 0.01 mg/kg to about 500 mg/kg per day, such as from about 0.01 mg/kg to about 400 mg/kg per day, such as about 0.01 mg/kg to about 200 mg/kg per day, such as about 0.1 mg/kg to about 100 mg/kg per day, such as about 0.01 mg/kg to about 10 mg/kg per day, such as about 0.01 mg/kg to about 2 mg/kg per day, including about 0.02 mg/kg to about 2 mg/kg per day. In other embodiments, the dosage (*e.g*., oral dosage) may range from about 0.01 to 100 mg/kg four times per day (QID), such as about 0.01 to about 50 mg/kg QID, such as about 0.01 mg/kg to about 10 mg/kg QID, such as 0.01 mg/kg to about 2 mg/kg QID, such as about 0.01 to about 0.2 mg/kg QID. In other embodiments, the dosage (*e.g.*, oral dosage) may range from about 0.01 mg/kg to about 50 mg/kg three times per day (TID), such as about 0.01 mg/kg to about 10 mg/kg TID, such as about 0.01 mg/kg to about 2 mg/kg TID, and including as about 0.01 mg/kg to about 0.2 mg/kg TID. In yet other embodiments, the dosage (*e.g*., oral dosage) may range from about 0.01 mg/kg to about 100 mg/kg two times per day (BID), such as about 0.01 mg/kg to about 10 mg/kg BID, such as about 0.01 mg/kg to about 2 mg/kg BID, including about 0.01 mg/kg to about 0.2 mg/kg BID. The amount of compound administered will depend on the potency and concentration of the specific NASSP, the magnitude or procoagulant effect desired, and the inherent absorptivity and/or bioavailability of the NASSP. Each of these factors is readily determined by one of skill in the art using the methods set forth herein or methods recognized in the art.

In various embodiments of the methods herein, the NASSP is orally administered in combination with one or more permeation enhancer. Appropriate permeation enhancers and their use with procoagulants, such as those provided by the present invention are disclosed in U.S. Provisional Patent Application No. 61/509,514. In some embodiments the permeation enhancer is a gastrointestinal epithelial barrier permeation enhancer. In various embodiments, the invention provides methods for enhancing blood coagulation by orally administering a composition including a procoagulant amount of a NASSP in combination with a gastrointestinal epithelial permeation enhancer to a subject. In various embodiments, the invention provides methods for enhancing blood coagulation by orally administering a composition including a procoagulant amount of a NASSP in combination with a gastrointestinal epithelial permeation enhancer and a blood coagulation factor to a subject.

In another aspect, the invention provides an *in vitro* method of inhibiting TFPI activity with a sufficient amount of a NASSP to inhibit TFPI activity. In certain embodiments, TFPI activity is inhibited in a subject by a method comprising administering a therapeutically effective amount of a composition comprising a NASSP to the subject. In certain embodiments, the invention provides a method of inhibiting TFPI activity in a biological sample, the method comprising combining the biological sample (*e.g*., blood or plasma) with a sufficient amount of a NASSP to inhibit TFPI activity.

In another aspect, the invention provides a method of inhibiting TFPI activity in a biological sample, the method comprising combining the biological sample (*e.g*., blood or plasma) with a sufficient amount of a non-anticoagulant sulfated or sulfonated synthetic polymer (NASSP) to inhibit TFPI activity.

In another aspect, the invention provides a method of measuring acceleration of clotting by a NASSP in a biological sample, the method comprising:
a) combining the biological sample with a composition comprising the NASSP,
b) measuring the clotting time of the biological sample,
c) comparing the clotting time of the biological sample to the clotting time of a corresponding biological sample not exposed to the NASSP, wherein a decrease in the clotting time of the biological sample exposed to the NASSP, if observed, is indicative of a NASSP that accelerates clotting.

Below are examples of specific embodiments for carrying out the present invention. The examples are offered for illustrative purposes only, and are not intended to limit the scope of the present invention in any way.

### EXAMPLES

It is understood that the examples and embodiments described herein are for illustrative purposes only and that various modifications or changes in light thereof will be suggested to persons skilled in the art and are to be included within the spirit and purview of this application and scope of the appended claims. All publications, patents, and patent applications cited herein are hereby incorporated by reference in their entirety for all purposes.

### EXAMPLE 1

As an extension of this class of compounds, sulfated or sulfonated, non-carbohydrate polymers (**FIG. 3**) are disclosed here. The present invention provides a method and composition suitable for treating bleeding disorders.

The procoagulant activity of these sulfated or sulfonated, non-carbohydrate based polymers was assessed by the Thrombin Generation Assay (TGA). The influence of each sulfated polymer on thrombin generation was measured in duplicate via CAT in a Fluoroskan Ascent® reader (Thermo Labsystems, Helsinki, Finland; filters 390 nm excitation and 460 nm emission) following the slow cleavage of the fluorogenic substrate Z-Gly-Gly-Arg-AMC (Hemker HC. Pathophysiol Haemost Thromb 2003; 33: 4 15). To each well of a 96-well microplate (Immulon 2HB, clear U-bottom; Thermo Electron) 80 µL of pre-warmed (37°C) goat anti-FVIII antibody treated human normal plasma pool was added. For triggering thrombin generation by tissue factor, 10 µL of PPP reagent containing a certain amount of recombinant human tissue factor (rTF) and phospholipid vesicles composed of phosphatidylserine, phosphatidylcholine and phosphatidylethanolamine (48 µM) (Thrombinoscope BV, Maastricht, The Netherlands) was added. For studying the procoagulant activity of sulfated polymers a final rTF concentration of 1 pM was used to provide FVIII and Tissue Factor Pathway Inhibitor (TFPI) sensitivity of the test system. In some experiments, TFPI activity was blocked in presence or absence of NASSPs by either a polyclonal goat anti-human TFPI antibody (R&D Systems, Af2974, Minneapolis, US) or a monoclonal anti-TFPI antibody directed against the positively charged C-terminus of TFPI (Sanquin White Label Products, MW1848, clone CLB/TFPI C-terminus, Amsterdam, The Netherlands) at plasma concentration of 25 nM or 50 nM, respectively. In addition, plasma treated with monoclonal anti-TFPI C-terminus antibody was supplemented with recombinant C-terminally truncated TFPI (aa1-160). Just prior to putting the plate into the pre-warmed (37°C) reader, 10 µL of test or reference sample or calibrator compound was added. Thrombin generation was started by dispensing 20 µL of FluCa reagent (Thrombinoscope BV, Maastricht, The Netherlands) containing fluorogenic substrate and Hepes buffered CaCl₂ (100 mM) into each well and fluorescence intensity was recorded at 37°C.

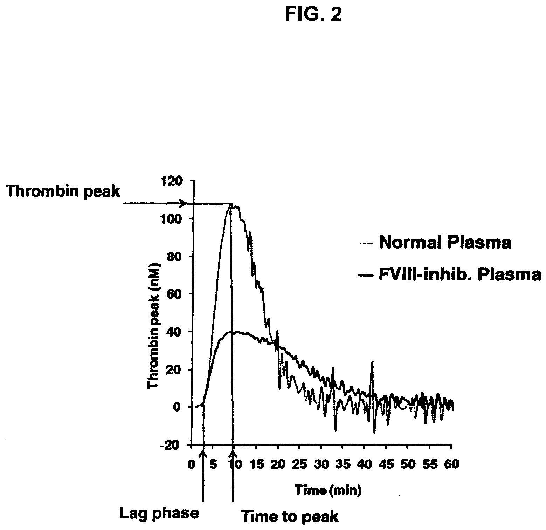

The parameters of the resulting thrombin generation curves were calculated using the Thrombinoscope™ software (Thrombinoscope BV, Maastricht, The Netherlands) and thrombin calibrator to correct for inner filter and substrate consumption effects (Hemker HC, Pathophysiol Haemost Thromb 2003; 33(4):15). With the thrombin calibrator as a reference, the molar concentration of thrombin in the test wells was calculated by the software. The thrombin amounts at the peak and peak times of each thrombin generation curve (peak thrombin, nM) were plotted against sulfated polymer concentrations resulting in the procoagulant profile of these compounds (**FIG. 2**). The thrombin generation assay results are illustrated in **FIGS. 4A****,** **4B****,** **4C****, and** **4E****.** NASSPs are procoagulant in a broad concentration range spanning at least two orders of magnitude starting at about 1 µg/mL, whereas a phosphorylated polymer is essentially inactive providing evidence for the importance of negatively charged sulfate or sulfonate groups (**FIG 4D**). At concentrations of optimal procoagulant activity (30 to 100 µg/mL) NASSPs exceeded the thrombin generation of a human normal plasma pool. At concentrations higher than 100 µg/mL sulfated polymers prolonged the activated partial thromboplastin time which is indicative of their anticoagulant activity.

Dilute prothrombin time assay with TFPI. A dilute prothrombin time assay with added tissue factor pathway inhibitor (TFPI-dPT) was used to evaluate the TFPI-inhibiting effect of the different NASSPs. Pooled normal human plasma (George King Biomedical, Overland Park, KS) was pre-incubated with 0.5 µg/mL full-length TFPI (aa 1-276, constitutively produced by SKHep1) and the respective NASSP (0 - 1000 µg/mL) for 15 min at RT. TF reagent TriniClot PT Excel S (Trinity Biotech, Wicklow, Ireland), diluted in Hepes-buffered saline 1:200 with 0.5 % BSA was added to the plasma samples on an ACL Pro Elite hemostasis analyzer (Instrumentation Laboratory, Bedford, MA). Clotting was initiated with 25 mM CaCl₂. The volume ratio of plasma:TF:CaCl₂ was 1:1:1 For data analysis, TFPI- dPT is plotted against the log concentration. Half maximal effective concentrations (EC50) values are determined using a sigmoidal curve fit.

Phosphorylated polymers analogues (*i.e*., polyvinyl phosphonate, polyvinyl phosphate) demonstrated no procoagulant effect (**FIG. 4D**). Because phosphate and sulfate groups are chemically very similar, this result was surprising. In addition there is literature on the procoagulant activity of polyphosphates: Müller et al, Cell, 139, 1143-1156; 2009.

### EXAMPLE 2

### Activated partial thromboplastin time assay (aPTT)

The aPTT assay was performed to study anticoagulant activities of NASSP. In brief, 50 µL of thawed normal human plasma pool (George King Biomedical, Overland Park, KS) was mixed with 5 µL of NASSPs (0-500 µg/mL final plasma concentration). NASSPs were diluted in imidazole buffer (3.4 g/L imidazole; 5.85 g/L NaCl, pH 7.4) containing 1% albumin (Baxter, Austria). After addition of 50 µL aPTT reagent (STA APTT, Roche) the samples were incubated for 4 min at 37 °C. Clotting was initiated by 50 µL 25 mM CaCl2 solution (Baxter, Austria) and recorded for up to 5 min. All pipetting steps and clotting time measurements were carried out with an ACL Pro Elite (Instrumentation Laboratory, Bedford, MA) instrument. Samples were run in duplicate.

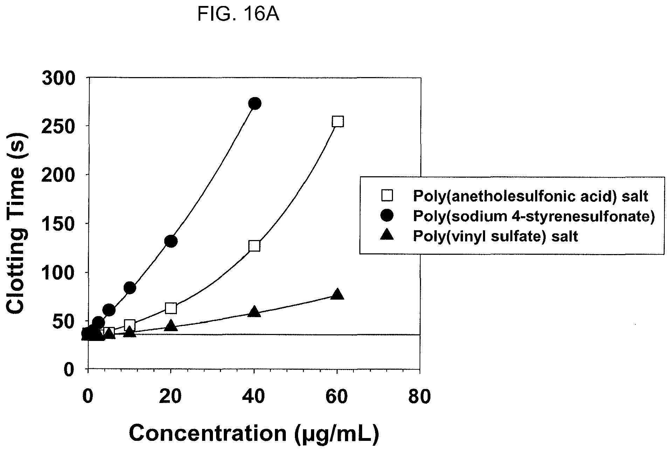

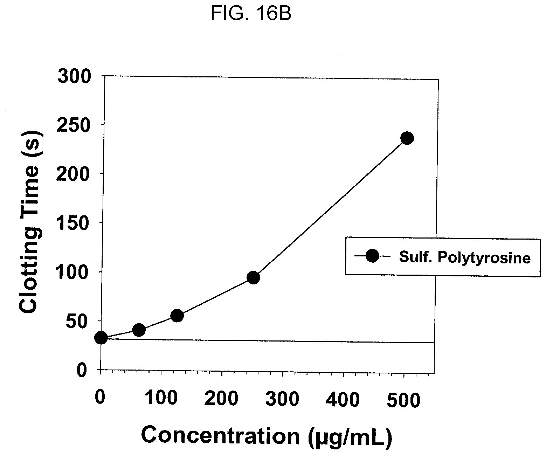

For data analysis, clotting time is plotted against the NASSP concentration. Concentrations where the clotting time is 50% increased over the normal plasma control are determined using a linear curve fit. See, **FIG. 16****.**

### EXAMPLE 3

### Caco-2 cell/ In-vivo Studies

### Objective:

One strategy to improve the oral bioavailability of NASSPs is the application of tight-junction-modulating permeation enhancers such as chitosan, bromelain, deoxycholine (DOC), or sodium caprate. The goal of this study is to determine the *in-vitro* resorption of selected NASSPs in the Caco-2-cell model in the absence and presence of permeation enhancers.

### Methods:

Human colon adenocarcinoma (Caco-2) cells cultured on semi-permeable filters spontaneously differentiate to form a confluent monolayer. This cell layer resembles both, structurally and functionally, the small intestinal epithelium. Caco-2 cells are cultured in a PET transwell-24 plate in RPMI-cell growth medium supplemented with 10% fetal calf serum and 1% L-glutamine. After 21 days in an incubator at 37°C and 95% air, 5% CO₂ atmosphere, a confluent monolayer is obtained. A selected NASSP dissolved in 200 µL growth medium with or without permeation enhancers is added onto the cells in the apical compartment at a concentration of 1 mg/mL and incubated at 37 °C. Medium samples (100 µL) are collected at 2, 4, 6 and 8 h from the basolateral side (850 µL volume) and before and at 8 h from the apical side. At each sample collection, the removed aliquot is replaced with fresh growth medium. To ensure that the cell layer stays intact during the experiment, the transepithelial electric resistance (TEER) is monitored and recorded. In each experiment, triplicate wells are tested and the experiment performed one to three times.

The amount of NASSP that is transferred from the apical into the basolateral compartment over a time period of 8 h is determined by a semi-quantitative activity-based thrombin generation assay (CAT) for all time points and a substance-specific liquid chromatography mass spectroscopy for the 8 h time point only.

### Results:

The resorption of a synthetic NASSP in combination with 4 or 5 enhancers is studied in the Caco-2 cell model. The amount of NASP on the basolateral side of the cells increases with time. The theoretically possible maximal concentration (µg/mL) is slightly different for each time point due to the dilution factor caused by the sampling. The possible maximum is expressed as µg/mL NASSP. The resorption at the 8 h time point is also expressed in % resorption Resorption in the presence of enhancers increases.

### EXAMPLE 4

### Objective:

To study the efficacy of a NASSP in an *ex-vivo* whole blood TEG FVIII-inhibited guinea pig model in improving clotting parameters.

### Methods:

Male Dunkin Hartley guinea pigs are intravenously injected with a goat anti-human FVIII inhibitor plasma at a dose of 42 BU/kg (1.9 mL/kg) 45 min before sampling. After 40 min, NASSP is intravenously administered to the animals at 0.05, 0.15, 0.45 or 1.35 mg/kg (N=5 per group). 300 U/kg FEIBA (Baxter, BioScience, Austria) serves as a positive control and saline as a vehicle control. Shortly after the injection, the *Vena cava* is punctured and blood is collected in the presence of citrate (ratio 1:9) for whole blood TEG analysis. Measurements are performed using a thromboelastography (TEG) hemostasis analyzer 5000 (Haemonetics Corp, USA) at 37°C. Every blood sample is prepared by pre-warming 20 µL of a 0.2 M CaCl₂ solution in a TEG cuvette at 37°C adding 340 µL of blood, mixing, and then immediately starting the TEG recording. The measurement proceeded for at least 120 min. The TEG parameters of clotting time (R-time), rapidity of clot strengthening (angle) and maximum clot firmness (MA) are recorded. The primary endpoint R-time is plotted and the median value of the different dosing groups compared with each other.

### Results:

Based on *in vitro* results from CAT assays with aNASSP, a dosage pattern for studying the procoagulant effect in FVIII-inhibited guinea pigs (n=5) after intravenous administration of 0.05; 0.15, 0.45 or 1.35 mg/kg NASSP is utilized. The animals show a slightly reduced median R-time (clotting time) when dosed with 0.15 and 0.45 mg/kg NASSP.

### EXAMPLE 5

### Objective:

To study the pharmacokinetic properties of a NASSP in CD rats after oral administration. The study also addresses the question whether a permeation enhancer improves *in vivo* oral bioavailability of a NASSP.

### Methods:

After a night of fasting, male CD rats are orally gavaged with two liquid preparations of NASSP at a dose of 50 mg/kg and 5 mL/kg. The substances are prepared in a physiological saline solution without enhancer or with 0.8 wt% DOC or with 3 wt% chitosan+0.5 mg/mL bromelain. Each group undergoing dosing with NASSP consisted of six rats. For each formulation, three additional rats serve as vehicle controls. Blood samples are collected in the presence of citrate (ratio 1:9) before dosing and 15, 30 min, 1, 5, and 7 h after the dosing. Platelet-poor plasma is prepared by two centrifugation steps at 3000 rpm for 10 min.

The NASSPs in the plasma samples are detected by a fluorescence assay. Experiments are performed in a black half-area 96-well microtiter plate (Costar).

### Results:

Rats orally dosed with 50 mg/kg NASSP in saline show detectable plasma levels of NASSP.

### EXAMPLE 6

### Objective:

To study the pharmacokinetic properties of a NASSP in CD rats after intravenous administration.

### Methods:

Male CD rats are intravenously administered with a NASSP at a dose of 5 mg/kg. The substances are prepared in a physiological saline solution and injected at 5 mL/kg. Each group consisted of three animals. Blood sample are collected in the presence of citrate (ratio 1:9) before dosing and 5, 30 min, 1, 3, 6, and 10 h after the dosing. Platelet-poor plasma is prepared by two centrifugation steps at 3000 rpm. The plasma samples are analyzed for NASSP by liquid-chromatography -mass spectroscopy.

It is understood that the examples and embodiments described herein are for illustrative purposes only and that various modifications or changes in light thereof will be suggested to persons skilled in the art and are to be included within the spirit and purview of this application and scope of the appended claims. All publications, patents, and patent applications cited herein are hereby incorporated by reference in their entirety for all purposes.

The present invention is also exemplified by the following preferred embodiments:
**1.** A pharmaceutical composition comprising a therapeutically effective amount of a non-anticoagulant, non-saccharide, sulfonated/sulfated synthetic polymer (NASSP); and a pharmaceutically acceptable excipient.
**2.** The composition according to item 1, wherein the polymer has a formula selected from: 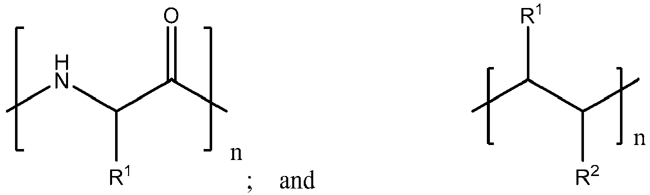 wherein
   R¹ is selected from H, substituted or unsubsituted alkyl and sulfonated aryl;
   R² is selected from H, substituted or unsubstituted alkyl, sulfonate and sulfate, such that at least one of R¹ and R² is a moiety which is a member selected from sulfonate and sulfate; and
   the index n represents an integer sufficient to provide a polymer of a molecular weight of from about 7 kDa to about 300 kDa.
**3.** The composition according to item 2, wherein
   R¹ is: 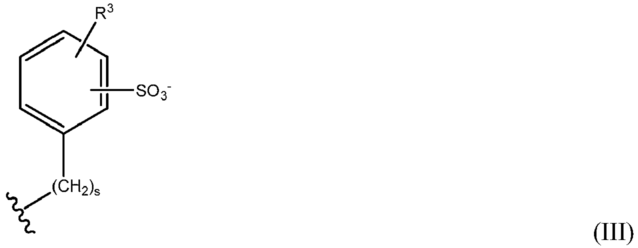 in which
   R³ is selected from H and OR⁴;
   R⁴ is selected from H, and substituted or unsubstituted alkyl; and
   s is an integer selected from 0, 1, 2, 3, 4 or greater.
**4.** A unit dosage formulation for use in a method for treating a subject in need of enhanced blood coagulation comprising administering a therapeutically effective amount of a composition comprising a non-anticoagulant, non-saccharide sulfonated/sulfated polymer to the subject, the unit dosage formulation comprising the polymer according to any of items 1-3 in a therapeutically effective amount.
**5.** The unit dosage formulation according to item 4, comprising from about 0.5 mg to about 1000 mg of the non-anticoagulant, non-saccharide sulfonated/sulfated polymer.
**6.** The unit dosage formulation according to item 4 or 5 wherein the polymer is in an amount sufficient to provide a dosage from about 0.01 mg/kg to about 100 mg/kg.
**7.** The unit dosage formulation according to any of items 4-6, wherein the polymer is present in the formulation in an amount sufficient to enhance blood coagulation in a subject to whom the unit dosage formulation is administered.
**8.** The unit dosage formulation according any of items 4-7, wherein the unit dosage formulation is an oral unit dosage formulation.
**9.** A method for treating a subject in need of enhanced blood coagulation, comprising administering a therapeutically effective amount of a composition comprising a non-anticoagulant, non-saccharide sulfonated/sulfated polymer to said subject.
**10.** The method according to item 9, wherein the polymer is administered to the subject at a dosage of about 0.01 mg/kg to about 100 mg/kg.
**11.** The method according to item 9 or 10, wherein the polymer is administered as a unit dosage formulation.
**12.** The method according to any of items 9-11, wherein the polymer is administered orally.
**13.** The method according to any of items 9-12, wherein the subject has a bleeding disorder selected from the group consisting of a chronic or acute bleeding disorder, a congenital coagulation disorder caused by a blood factor deficiency, and an acquired coagulation disorder.
**14.** The method according to item 17, wherein the blood factor deficiency is a deficiency of one or more factors selected from the group consisting of Factor V, Factor VII, Factor VIII, Factor IX, Factor XI, Factor XII, Factor XIII, and von Willebrand Factor.
**15.** The method according to any of items 9-12, wherein said polymer is administered to said subject prior to surgery or other invasive procedure.
**16.** The method according to any of items 7-15, further comprising administering an agent selected from the group consisting of a procoagulant, an activator of the intrinsic coagulation pathway, an activator of the extrinsic coagulation pathway, a non-anticoagulant sulfated/sulfonated polysaccharide (NASP), and a second non-anticoagulant, non-saccharide sulfonated/sulfated polymer, which has a structure different from the non-anticoagulant, non-saccharide sulfonated/sulfated polymer.
**17.** The method of item 16, wherein the agent is selected from the group consisting of tissue factor, Factor II, Factor V, Factor Va, Factor VII, Factor VIIa, Factor VIII, Factor VIIIa, Factor X, Factor X, Factor Xa, Factor IXa, Factor XI, Factor XIa, Factor XII, Factor XIIa, Factor XIII, prekallikrein, HMWK, and von Willebrand Factor.
**18.** The method of item 16, wherein the anticoagulant is selected from the group consisting of heparin, a coumarin derivative, such as warfarin or dicumarol, Tissue Factor Pathway Inhibitor (TFPI), antithrombin III, lupus anticoagulant, nematode anticoagulant peptide (NAPc2), active-site blocked Factor VIIa (Factor VIIai), Factor IXa inhibitors, Factor Xa inhibitors, including fondaparinux, idraparinux, DX-9065a, and razaxaban (DPC906), inhibitors of Factors Va and VIIIa, including activated protein C (APC) and soluble thrombomodulin, thrombin inhibitors, including hirudin, bivalirudin, argatroban, and ximelagatran, and an antibody that binds a coagulation factor.
**19.** The method of item 16, wherein the anticoagulant is an antibody that binds a coagulation factor selected from the group consisting of Factor V, Factor VII, Factor VIII, Factor IX, Factor X, Factor XIII, Factor II, Factor XI, Factor XII, von Willebrand Factor, prekallikrein, and HMWK.
**20.** A method of inhibiting Tissue Factor Pathway Inhibitor (TFPI) activity in a subject, the method comprising administering to the subject an amount of a composition comprising a non-anticoagulant, non-saccharide sulfonated/sulfated polymer to the subject sufficient to inhibit the TFPI.
**21.** A method of inhibiting TFPI activity in a biological sample, the method comprising combining the biological sample with a sufficient amount of a non-anticoagulant, non-saccharide sulfonated/sulfated polymer to inhibit the TFPI activity.
**22.** A method of measuring acceleration of blood clotting by a non-anticoagulant, non-saccharide, sulfonated/sulfated polymer in a biological sample, the method comprising: a) combining the biological sample with a composition comprising the polymer, b) measuring the clotting time of the biological sample, c) comparing the clotting time of the biological sample to the clotting time of a corresponding biological sample not exposed to the polymer, wherein a decrease in the clotting time of the biological sample exposed to the polymer is indicative of a polymer that accelerates the clotting.

## Claims

1. A pharmaceutical composition comprising a therapeutically effective amount of a non-anticoagulant, non-saccharide, sulfonated/sulfated synthetic polymer (NASSP); and a pharmaceutically acceptable excipient.

2. The composition according to claim 1, wherein the polymer has a formula selected from: 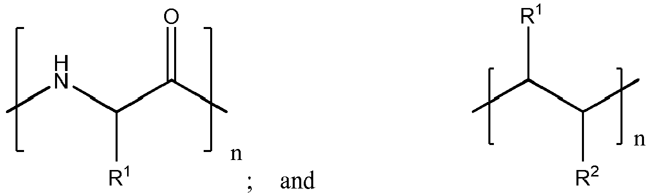 wherein
R¹ is selected from H, substituted or unsubsituted alkyl and sulfonated aryl;
R² is selected from H, substituted or unsubstituted alkyl, sulfonate and sulfate, such that at least one of R¹ and R² is a moiety which is a member selected from sulfonate and sulfate; and
the index n represents an integer sufficient to provide a polymer of a molecular weight of from about 7 kDa to about 300 kDa,
preferably wherein
R¹ is: 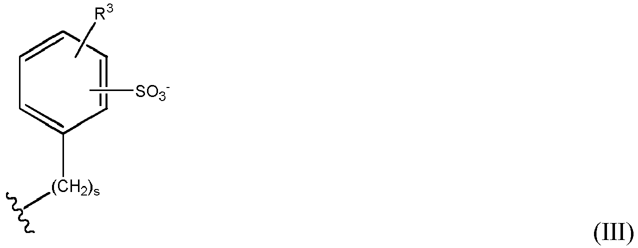 in which
R³ is selected from H and OR⁴;
R⁴ is selected from H, and substituted or unsubstituted alkyl; and
s is an integer selected from 0, 1, 2, 3, 4 or greater.

3. A unit dosage formulation for use in a method for treating a subject in need of enhanced blood coagulation comprising administering a therapeutically effective amount of a composition comprising a non-anticoagulant, non-saccharide sulfonated/sulfated polymer to the subject, the unit dosage formulation comprising the polymer according to any of claims 1-2 in a therapeutically effective amount.

4. The unit dosage formulation according to claim 3, wherein
**(i)** the unit dosage formulation comprises from about 0.5 mg to about 1000 mg of the non-anticoagulant, non-saccharide sulfonated/sulfated polymer,
**(ii)** the polymer is in an amount sufficient to provide a dosage from about 0.01 mg/kg to about 100 mg/kg,
**(iii)** the polymer is present in the formulation in an amount sufficient to enhance blood coagulation in a subject to whom the unit dosage formulation is administered, and/or
**(iv)** the unit dosage formulation is an oral unit dosage formulation.

5. A composition comprising a non-anticoagulant, non-saccharide sulfonated/sulfated polymer for use in a method for treating a subject in need of enhanced blood coagulation, the method comprising administering a therapeutically effective amount of the composition comprising the non-anticoagulant, non-saccharide sulfonated/sulfated polymer to said subject.

6. The composition according to claim 5 for the use according to claim 5, wherein
**(i)** the polymer is administered to the subject at a dosage of about 0.01 mg/kg to about 100 mg/kg,
**(ii)** the polymer is administered as a unit dosage formulation, and/or
**(iii)** the polymer is administered orally.

7. The composition according to any of claims 5-6 for the use according to any of claims 5-6, wherein the subject has a bleeding disorder selected from the group consisting of a chronic or acute bleeding disorder, a congenital coagulation disorder caused by a blood factor deficiency, and an acquired coagulation disorder.

8. The composition according to claim 7 for the use according to claim 7, wherein the blood factor deficiency is a deficiency of one or more factors selected from the group consisting of Factor V, Factor VII, Factor VIII, Factor IX, Factor XI, Factor XII, Factor XIII, and von Willebrand Factor.

9. The composition according to any of claims 5-6 for the use according to any of claims 5-6, wherein said polymer is administered to said subject prior to surgery or other invasive procedure.

10. The composition according to any of claims 5-9 for the use according to any of claims 5-9, the method further comprising administering an agent selected from the group consisting of a procoagulant, an activator of the intrinsic coagulation pathway, an activator of the extrinsic coagulation pathway, a non-anticoagulant sulfated/sulfonated polysaccharide (NASP), and a second non-anticoagulant, non-saccharide sulfonated/sulfated polymer, which has a structure different from the non-anticoagulant, non-saccharide sulfonated/sulfated polymer.

11. The composition of claim 10 for the use of claim 10, wherein
**(i)** the agent is selected from the group consisting of tissue factor, Factor II, Factor V, Factor Va, Factor VII, Factor VIIa, Factor VIII, Factor VIIIa, Factor X, Factor X, Factor Xa, Factor IXa, Factor XI, Factor XIa, Factor XII, Factor XIIa, Factor XIII, prekallikrein, HMWK, and von Willebrand Factor,
**(ii)** the anticoagulant is selected from the group consisting of heparin, a coumarin derivative, such as warfarin or dicumarol, Tissue Factor Pathway Inhibitor (TFPI), antithrombin III, lupus anticoagulant, nematode anticoagulant peptide (NAPc2), active-site blocked Factor VIIa (Factor VIlai), Factor IXa inhibitors, Factor Xa inhibitors, including fondaparinux, idraparinux, DX-9065a, and razaxaban (DPC906), inhibitors of Factors Va and VIIIa, including activated protein C (APC) and soluble thrombomodulin, thrombin inhibitors, including hirudin, bivalirudin, argatroban, and ximelagatran, and an antibody that binds a coagulation factor, or
**(iii)** the anticoagulant is an antibody that binds a coagulation factor selected from the group consisting of Factor V, Factor VII, Factor VIII, Factor IX, Factor X, Factor XIII, Factor II, Factor XI, Factor XII, von Willebrand Factor, prekallikrein, and HMWK.

12. A composition comprising a non-anticoagulant, non-saccharide sulfonated/sulfated polymer for use in a method of inhibiting Tissue Factor Pathway Inhibitor (TFPI) activity in a subject, the method comprising administering to the subject an amount of the composition comprising the non-anticoagulant, non-saccharide sulfonated/sulfated polymer sufficient to inhibit the TFPI.

13. A method of inhibiting TFPI activity in a biological sample, the method comprising combining the biological sample with a sufficient amount of a non-anticoagulant, non-saccharide sulfonated/sulfated polymer to inhibit the TFPI activity.

14. A method of measuring acceleration of blood clotting by a non-anticoagulant, non-saccharide, sulfonated/sulfated polymer in a biological sample, the method comprising: a) combining the biological sample with a composition comprising the polymer, b) measuring the clotting time of the biological sample, c) comparing the clotting time of the biological sample to the clotting time of a corresponding biological sample not exposed to the polymer, wherein a decrease in the clotting time of the biological sample exposed to the polymer is indicative of a polymer that accelerates the clotting.
